# EUROPEAN PATENT APPLICATION

(11) **EP 4 851 159 A1**
(43) Date of publication of application: **09.12.2026**
(21) Application number: 25747825.5
(22) Date of filing: 24.01.2025
(51) Int. Cl.: C07D 413/14, C07D 405/14, C07D 405/12, C07D 405/04, A61K 31/496, A61P 3/10

(54) **METHOD FOR PREPARING BENZODIOXANE OR SALTS THEREOF**

(30) Priority: 01.02.2024 CN 202410142327
(71) Applicant: Shanghai Senhui Medicine Co., Ltd., Shanghai 201203 (CN)
(72) Inventor: JIANG, Wei, Shanghai 201203 (CN); HUANG, Jian, Shanghai 201203 (CN); LUO, Guangshun, Shanghai 201203 (CN); GAO, Junfeng, Shanghai 201203 (CN); LI, Bei, Shanghai 201203 (CN); ZHOU, Yinan, Shanghai 201203 (CN)
(74) Representative: J A Kemp LLP
(86) International application number: PCT/CN2025/074695
(87) International publication number: WO 2025/162257

(57) **Abstract**

The present disclosure relates to a method for preparing a benzodioxane or salts thereof. Specifically, the method of the present disclosure comprises a step of reacting a compound of formula E under the presence of a tri-substituted phosphine/azodicarboxylate to form a compound of formula F.

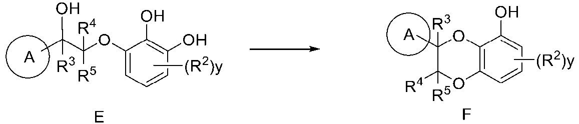

## Description

### TECHNICAL FIELD

The present disclosure belongs to the field of medicine, and relates to a method for preparing benzodioxane or salts thereof.

### BACKGROUND

4-(3-(4-chloro-2-fluorophenyl)-2,3-dihydrobenzo[b][1,4]dioxan-5-yl)piperidine is a pharmaceutical intermediate and a fragment of multiple new drugs in research, such as the active molecule PF06882961 reported in WO2018109607 for the treatment of obesity.

WO2022007979 discloses a synthesis method for 4-(3-(4-chloro-2-fluorophenyl)-2,3-dihydrobenzo[b][1,4]dioxan-5-yl)piperidine. The method has low atom utilization ratio and low yield, and is not suitable for industrial production.

### SUMMARY

The present disclosure provides a method for preparing a compound of formula K or a salt thereof, 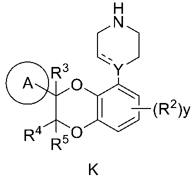 the method comprises a step of reacting a compound of formula E in the presence of trisubstituted phosphine/azodicarboxylate to form a compound of formula F, 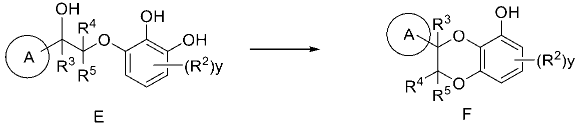 wherein,
ring A is selected from 6- to 10-membered aryl or 5- to 10-membered heteroaryl, wherein the aryl or heteroaryl is optionally substituted with one or more halogen, cyano, C₁₋₆ alkyl or C₁₋₆ alkoxy;
"-̅ -̅ -̅" is a double bond or a single bond,
when " --" is a single bond, Y is selected from N or CR¹⁴,
when "-̅ -̅ -̅" is a double bond, Y is CR¹⁴;
R² is selected from halogen, cyano, C₁₋₆ alkyl or C₁₋₆ alkoxy;
R³, R⁴, and R⁵ are each independently selected from hydrogen, halogen, cyano, C₁₋₆ alkyl or C₁₋₆ alkoxy, wherein the alkyl or alkoxy is optionally substituted with one or more halogen, hydroxyl, cyano, or amino;
R¹⁴ is selected from hydrogen, halogen, cyano, C₁₋₆ alkyl or C₁₋₆ alkoxy, wherein the alkyl or alkoxy is optionally substituted with one or more halogen, hydroxyl, cyano, or amino;
y is selected from 0, 1, 2, or 3.

In some embodiments, the trisubstituted phosphine is triphenylphosphine.

In some embodiments, the azodicarboxylate is selected from diethyl azodicarboxylate, diisopropyl azodicarboxylate, or di(4-chlorobenzyl) azodicarboxylate. In certain embodiments, the azodicarboxylate is diisopropyl azodicarboxylate.

In some embodiments, the reaction solvent for the compound of formula E is selected from tetrahydrofuran, diethyl ether, dichloromethane, toluene, ethyl acetate, acetonitrile, and N,N-dimethylformamide. In certain embodiments, the reaction solvent for the compound of formula E is dichloromethane.

In some embodiments, the molar ratio of the compound of formula E to the trisubstituted phosphine is 1:1 to 1:2, including but not limited to 1:1, 1:1.1, 1:1.2, 1:1.3, 1:1.4, 1:1.5, 1:1.6, 1:1.7, 1:1.8, 1:1.9, 1:2 or any value between any two numbers. In certain embodiments, the molar ratio of the compound of formula E to the trisubstituted phosphine is 1:1 to 1:1.5.

In certain embodiments, the molar ratio of the compound of formula E to the triphenylphosphine is 1:1 to 1:1.5.

In some embodiments, the molar ratio of the compound of formula E to the azodicarboxylate is 1:1 to 1:2, including but not limited to 1:1, 1:1.1, 1:1.2, 1:1.3, 1:1.4, 1:1.5, 1:1.6, 1:1.7, 1:1.8, 1:1.9, 1:2, or any value between any two numbers. In certain embodiments, the molar ratio of the compound of formula E to the azodicarboxylate is 1:1 to 1:1.5.

In certain embodiments, the molar ratio of the compound of formula E to the diisopropyl azodicarboxylate is 1:1 to 1:1.5.

In some embodiments, ring A is selected from 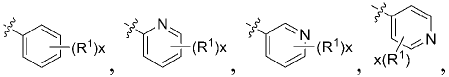 or 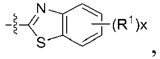 R¹ is selected from fluoro, chloro, bromo, iodo, or cyano, and x is selected from 0, 1, 2, 3, 4.

In certain embodiments, ring A is selected from 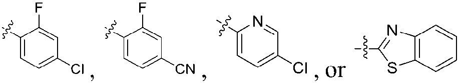 . In certain embodiments, ring A is

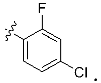

In some embodiments, the compound of formula K is the compound of formula K-1, 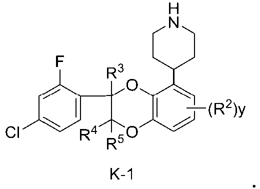 The method for preparing the compound of formula K-1 or a salt thereof comprises a step of reacting the compound of formula E-1 in the presence of trisubstituted phosphine/azodicarboxylate to form the compound of formula F-1, 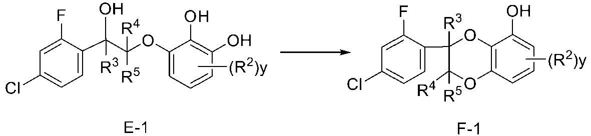 wherein R², R³, R⁴, R⁵, R⁶, R⁸, and y are as defined above.

In some embodiments, y is 0.

In some embodiments, the compound of formula K is the compound of formula K-2, 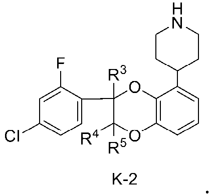 The method for preparing the compound of formula K-2 or a salt thereof comprises a step of reacting the compound of formula E-2 in the presence of trisubstituted phosphine/azodicarboxylate to form the compound of formula F-2, 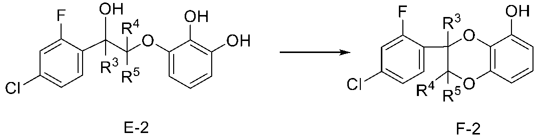 wherein R³, R⁴, and R⁵ are as defined above.

In some embodiments, R⁴ and R⁵ are each independently selected from hydrogen, methyl, ethyl, propyl, butyl, fluoro, chloro, bromo, and cyano. In certain specific embodiments, R⁴ and R⁵ are each independently hydrogen.

In some embodiments, the compound of formula K is the compound of formula K-3, 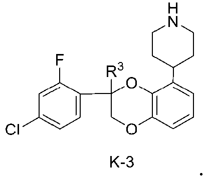 The method for preparing the compound of formula K-3 or a salt thereof comprises a step of reacting the compound of formula E-3 in the presence of trisubstituted phosphine/azodicarboxylate to form the compound of formula F-3, 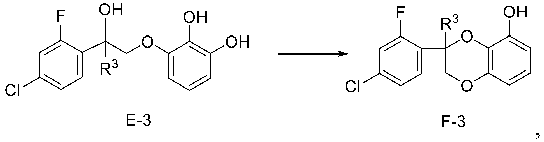 wherein, R³ is as defined above.

In some embodiments, R³ is selected from hydrogen, methyl, ethyl, propyl, butyl, fluoro, chloro, bromo, and cyano. In certain specific embodiments, R³ is selected from hydrogen and methyl.

In some embodiments, the compound of formula K is the compound of formula K-4, 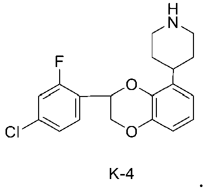 The method for preparing the compound of formula K-4 or a salt thereof comprises a step of reacting a compound of formula E-4 in the presence of triphenylphosphine/diisopropyl azodicarboxylate to form a compound of formula F-4,

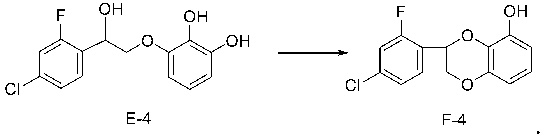

In some embodiments, the compound of formula K is the compound of formula K-4a, 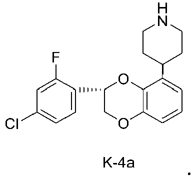 The method for preparing the compound of formula K-4a or a salt thereof comprises a step of reacting a compound of formula E-4a in the presence of triphenylphosphine/diisopropyl azodicarboxylate to form the compound of formula F-4a,

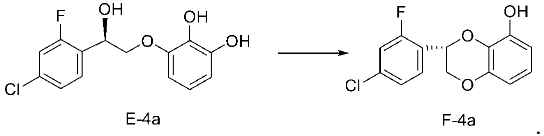

Further, the method of the present disclosure for preparing the compound of formula K or a salt thereof further comprises a step of converting the compound of formula D to a compound of formula M in the presence of an acid, and a step of converting the compound of formula M to the compound of formula E, 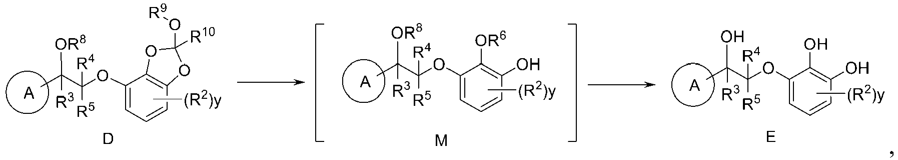 wherein,
R⁶ and R⁸ are each independently selected from hydrogen or a hydroxyl protecting group;
R⁹ and R¹⁰ are each independently selected from hydrogen, C₁₋₆ alkyl or C₁₋₆ alkoxy, wherein the alkyl or alkoxy is optionally substituted with one or more halogen, hydroxyl, cyano, or amino;
Ring A, R², R³, R⁴, R⁵, and y are as defined above.

In some embodiments, the acid is selected from hydrochloric acid, sulfuric acid, formic acid, acetic acid, and p-toluenesulfonic acid. In certain embodiments, the acid is hydrochloric acid.

In some embodiments, the reaction solvent for the compound of formula D is an alcohol. In some embodiments, the alcohol is selected from methanol, ethanol, propanol, butanol, pentanol, and hexanol. In certain embodiments, the alcohol is methanol.

In some embodiments, the compound of formula K is the compound of formula K-1. The method for preparing the compound of formula K-1 or a salt thereof further comprises a step of converting the compound of formula D-1 to the compound of formula M-1 in the presence of an acid, and a step of converting the compound of formula M-1 to the compound of formula E-1, 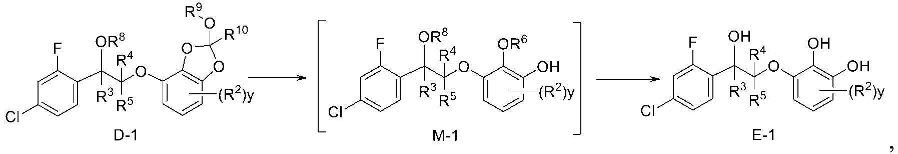 wherein, R⁹, R¹⁰, R², R³, R⁴, R⁵, R⁶, R⁸, and y are as defined above.

In some embodiments, the compound of formula K is the compound of formula K-2. The method for preparing the compound of formula K-2 or a salt thereof further comprises a step of converting the compound of formula D-2 to the compound of formula M-2 in the presence of an acid, and a step of converting the compound of formula M-2 to the compound of formula E-2, 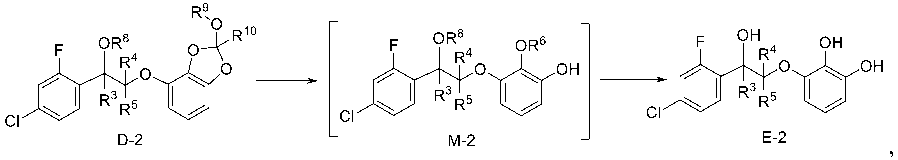 wherein, R⁶, R⁸, R⁹, R¹⁰, R³, R⁴, and R⁵ are as defined above.

In some embodiments, the compound of formula K is the compound of formula K-3. The method for preparing the compound of formula K-3 or a salt thereof further comprises a step of converting the compound of formula D-3 to the compound of formula M-3 in the presence of an acid, and a step of converting the compound of formula M-3 to the compound of formula E-3, 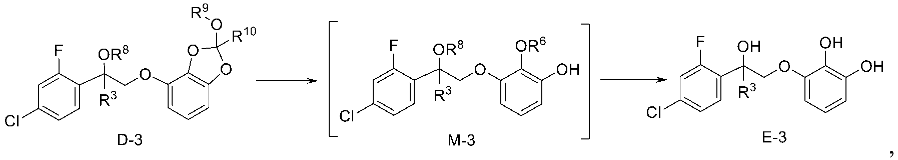 wherein, R⁶, R⁸, R⁹, R¹⁰, and R³ are as defined above.

In some embodiments, R⁹ is selected from hydrogen, methyl, ethyl, propyl, butyl, and isopropyl. In certain embodiments, R⁹ is ethyl.

In some embodiments, R¹⁰ is selected from hydrogen, methyl, ethyl, propyl, butyl, and isopropyl. In certain embodiments, R¹⁰ is methyl.

In some embodiments, the compound of formula K is the compound of formula K-4. The method for preparing the compound of formula K-4 or a salt thereof further comprises a step of converting the compound of formula D-4 to the compound of formula M-4 in the presence of hydrochloric acid/methanol, and a step of converting the compound of formula M-4 to the compound of formula E-4, 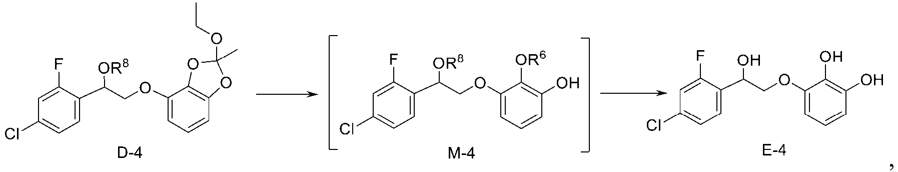 wherein, R⁶ and R⁸ are as defined above.

In some embodiments, the compound of formula K is the compound of formula K-4a. The method for preparing the compound of formula K-4a or a salt thereof further comprises a step of converting the compound of formula D-4a to a compound of formula M-4a in the presence of hydrochloric acid/methanol, and comprises a step of converting the compound of formula M-4a to the compound of formula E-4a, 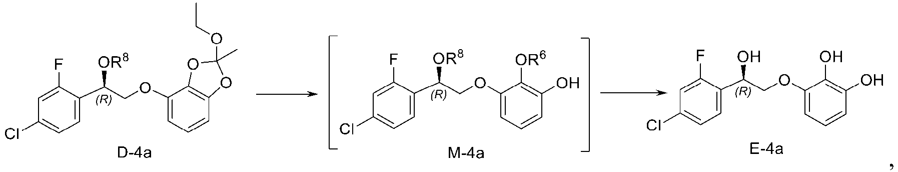 wherein, R⁶ and R⁸ are as defined above.

Further, the method of the present disclosure for preparing the compound of formula K or a salt thereof further comprises a step of forming the compound of formula D from the compound of formula C in the presence of an oxazaborolidine catalyst of formula (I)/a reducing agent, 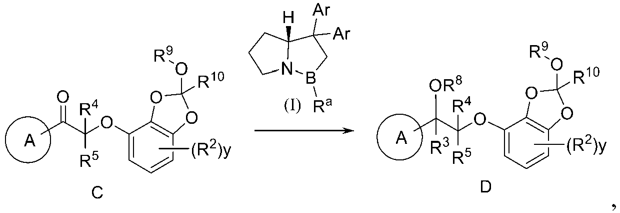
wherein: each Ar is independently selected from 6-membered aryl or 5- to 10-membered heteroaryl, wherein the aryl or heteroaryl is optionally substituted with one or more halogen, cyano, C₁₋₆ alkyl, or C₁₋₆ alkoxy;
R^{a} is selected from hydrogen or C₁₋₆ alkyl, wherein the alkyl is optionally substituted with one or more halogen, hydroxyl, cyano, or amino;
ring A, R⁸, R⁹, R¹⁰, R², R³, R⁴, R⁵, and y are as defined above.

In certain embodiments, each Ar is independently phenyl.

In certain embodiments, R^{a} is C₁₋₆ alkyl. In certain embodiments, R^{a} is methyl, ethyl, n-propyl, or isopropyl. In certain embodiments, R^{a} is methyl.

In certain embodiments, the oxazaborolidine catalyst of formula (I) is represented by formula (Ia)

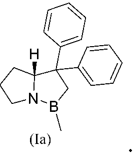

In some embodiments, the reducing agent is a borane-ligand compound. In certain specific embodiments, the reducing agent is BH₃·THF, BH₃·Me₂S, or BH₃·DEA. In certain specific embodiments, the reducing agent is BH₃·THF.

In some embodiments, the compound of formula K is the compound of formula K-1. The method for preparing the compound of formula K-1 or a salt thereof further comprises a step of forming the compound of formula D-1 from the compound of formula C-1 in the presence of an oxazaborolidine catalyst of formula (I)/a reducing agent, 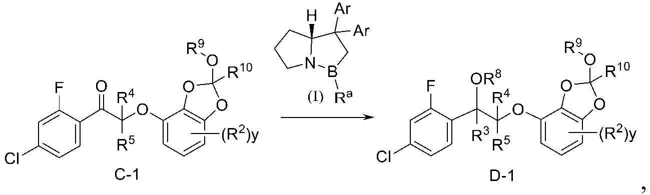 wherein Ar, R^{a}, R⁸, R⁹, R¹⁰, R², R³, R⁴, R⁵, and y are as defined above.

In some embodiments, the compound of formula K is the compound of formula K-2. The method for preparing the compound of formula K-2 or a salt thereof further comprises a step of forming the compound of formula D-2 from the compound of formula C-2 in the presence of an oxazaborolidine catalyst of formula (I)/a reducing agent, 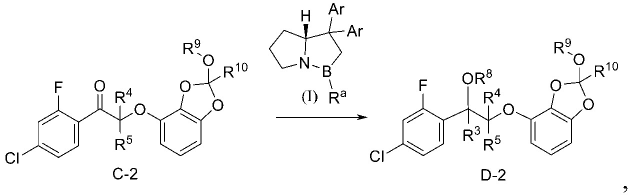 wherein Ar, R^{a}, R⁸, R⁹, R¹⁰, R², R³, R⁴, R⁵, and y are as defined above.

In some embodiments, the compound of formula K is the compound of formula K-3. The method for preparing the compound of formula K-3 or a salt thereof further comprises a step of forming the compound of formula D-3 from the compound of formula C-3 in the presence of an oxazaborolidine catalyst of formula (I)/a reducing agent, 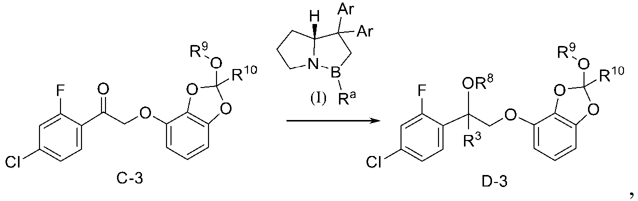 wherein Ar, R^{a}, R⁸, R⁹, R¹⁰, and R³ are as defined above.

In some embodiments, the compound of formula K is the compound of formula K-4. The method for preparing the compound of formula K-4 or a salt thereof further comprises a step of forming the compound of formula D-4 from the compound of formula C-4 in the presence of an oxazaborolidine catalyst of formula (Ia)/a reducing agent, 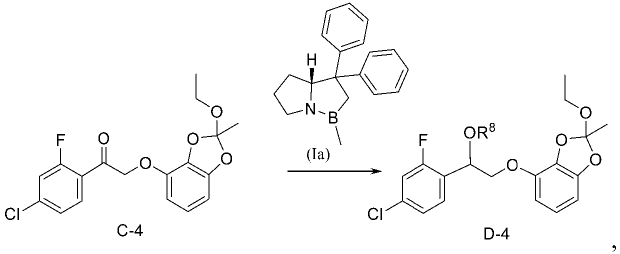 wherein R⁸ is as defined above.

In some embodiments, the compound of formula K is the compound of formula K-4a. The method for preparing the compound of formula K-4a or a salt thereof further comprises a step of forming the compound of formula D-4a from the compound of formula C-4 in the presence of an oxazaborolidine catalyst of formula (Ia)/BH₃·THF,

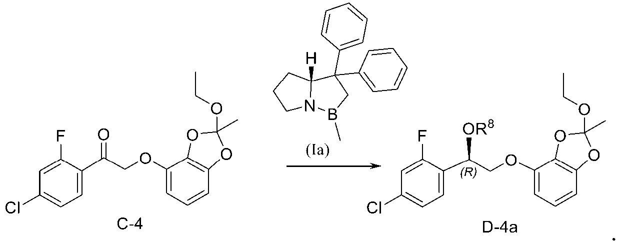

Further, the method of the present disclosure for preparing the compound of formula K or a salt thereof further comprises the steps of reacting the compound of formula (II) with the orthoester of formula (III) to form the compound of formula A, and reacting the compound of formula A with the compound of formula B in the presence of a base to form the compound of formula C, 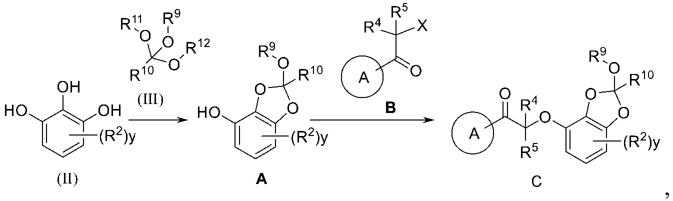
wherein: R¹¹ and R¹² are each independently selected from hydrogen, C₁₋₆ alkyl, or C₁₋₆ alkoxy, wherein the alkyl or alkoxy is optionally substituted with one or more halogen, hydroxyl, cyano, or amino;
X is halogen;
Ring A, R⁹, R¹⁰, R², R⁴, R⁵, and y are as defined above.

In some embodiments, the base is selected from potassium carbonate and sodium carbonate. In certain embodiments, the base is potassium carbonate.

In some embodiments, the compound of formula K is the compound of formula K-1. The method for preparing the compound of formula K-1 or a salt thereof further comprises the steps of reacting the compound of formula (II) with the orthoester of formula (III) to form the compound of formula A, and reacting the compound of formula A with the compound of formula B1 in the presence of a base to form the compound of formula C-1, 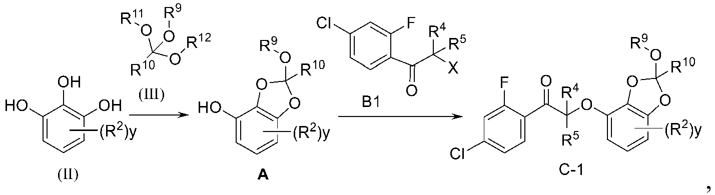 wherein: R⁹, R¹⁰, R¹¹, R¹², X, R², R⁴, R⁵, and y are as defined above.

In some embodiments, the compound of formula K is the compound of formula K-2. The method for preparing the compound of formula K-2 or a salt thereof further comprises the steps of reacting the compound of formula (IIa) with the orthoester of formula (III) to form a compound of formula A1, and reacting the compound of formula A1 with the compound of formula B1 in the presence of a base to form the compound of formula C-2, 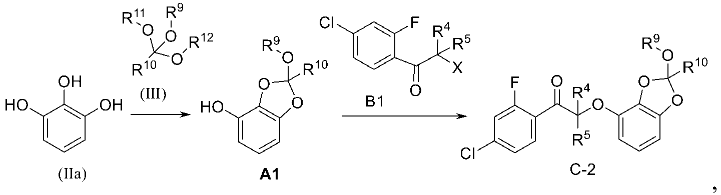 wherein: R⁹, R¹⁰, R¹¹, R¹², X, R⁴, and R⁵ are as defined above.

In some embodiments, the compound of formula K is the compound of formula K-3. The method for preparing the compound of formula K-3 or a salt thereof further comprises the steps of reacting the compound of formula (IIa) with the orthoester of formula (III) to form a compound of formula A1, and reacting the compound of formula A1 with the compound of formula B2 in the presence of a base to form the compound of formula C-3, 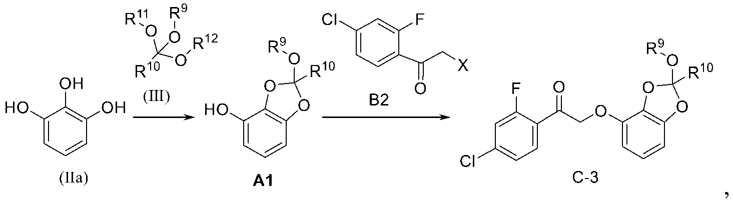 wherein: R⁹, R¹⁰, R¹¹, R¹², and X are as defined above.

In some embodiments, X is selected from fluoro, chloro, bromo, and iodo. In certain specific embodiments, X is bromo.

In some embodiments, R¹¹ and R¹² are each independently C₁₋₆ alkyl. In some embodiments, R¹¹ and R¹² are each independently selected from methyl, ethyl, propyl, butyl, and isopropyl. In certain specific embodiments, R¹¹ and R¹² are both ethyl.

In some embodiments, the compound of formula K is the compound of formula K-4. The method for preparing the compound of formula K-4 or a salt thereof further comprises the steps of reacting the compound of formula (IIa) with an orthoester of formula (IIIa) to form the compound of formula A2, and reacting the compound of formula A2 with the compound of formula B3 in the presence of potassium carbonate to form the compound of formula C-4,

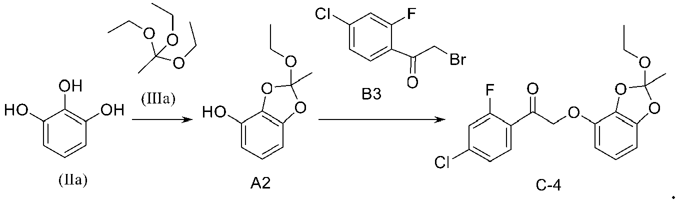

Further, the method of the present disclosure for preparing the compound of formula K or a salt thereof further comprises the steps of converting the compound of formula F into a compound of formula G, and reacting the compound of formula G with the compound of formula H in the presence of the palladium catalyst/co-catalyst to form the compound of formula J, 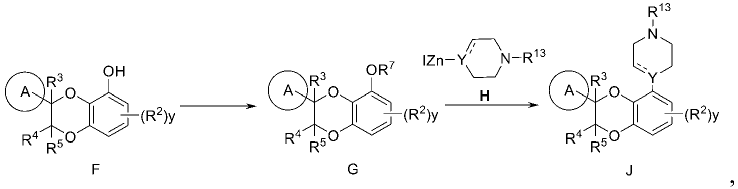
wherein: R⁷ is a hydroxyl protecting group; R¹³ is an amino protecting group;
ring A, R², R³, R⁴, R⁵, y, -̅ -̅ -̅, Y are as defined above.

In some embodiments, the hydroxyl protecting group is selected from allyl, methoxymethyl ether, tetrahydropyranyl, (trimethylsilyl)ethoxymethyl, dimethylaminosulfonyl, trifluoromethanesulfonyl, alkyl groups (e.g., methyl, tert-butyl, trityl, etc.), benzyl groups (e.g., benzyl, para-methoxybenzyl, etc.), silyl groups (e.g., tert-butyldimethylsilyl, etc.), acyl groups (e.g., acetyl, benzoyl, etc.), and alkoxycarbonyl groups (e.g., benzyloxycarbonyl, etc.). In certain embodiments, the hydroxyl protecting group is trifluoromethanesulfonyl. In certain embodiments, the R⁷ is trifluoromethanesulfonyl.

In some embodiments, the amino protecting group is selected from benzyloxycarbonyl, tert-butoxycarbonyl, 9-fluorenylmethoxycarbonyl, allyloxycarbonyl, 2-(trimethylsilyl)ethoxycarbonyl, para-toluenesulfonylmethoxycarbonyl, ethoxycarbonyl, phthaloyl, trifluoroacetyl, trityl, 2,4-dimethoxybenzyl, para-methoxybenzyl, and benzyl. In certain embodiments, the amino protecting group is tert-butoxycarbonyl. In certain embodiments, the R¹³ is tert-butoxycarbonyl.

In some embodiments, the palladium catalyst is a palladium-phosphine complex. In some embodiments, the palladium catalyst is selected from Pd(PPh₃)₄, Pd(PPh₃)₂Cl₂, Pd(dppe)Cl₂, Pd(dppp)Cl₂, Pd(dppf)Cl₂, a solvate of Pd(PPh₃)₄, a solvate of Pd(PPh₃)₂Cl₂, a solvate of Pd(dppe)Cl₂, a solvate of Pd(dppp)Cl₂, and a solvate of Pd(dppf)Cl₂. In some embodiments, the palladium catalyst is selected from Pd(dppf)Cl₂ or a solvate of Pd(dppf)Cl₂. In some embodiments, the solvate of Pd(dppf)Cl₂ is Pd(dppf)Cl₂·CH₂Cl₂.

In some embodiments, the palladium catalyst is Pd(dppf)Cl₂·CH₂Cl₂.

In some embodiments, the co-catalyst is selected from silver oxide or cuprous iodide.

In some embodiments, the solvent used for the reaction of the compound of formula G is selected from organic amines and diethyl ether. In some embodiments, the solvent used for the reaction of the compound of formula G is selected from triethylamine, ethylenediamine, dimethylformamide, and dimethylacetamide. In some specific embodiments, the solvent used for the reaction of the compound of formula G is dimethylacetamide.

In some embodiments, 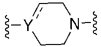 is selected from 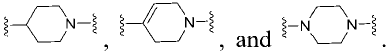 In certain embodiments, the 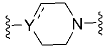 is

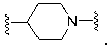

In some embodiments, the compound of formula K is the compound of formula K-1. The method for preparing the compound of formula K-1 or a salt thereof further comprises the steps of converting the compound of formula F-1 to the compound of formula G-1, and reacting the compound of formula G-1 with a compound of formula H-1 in the presence of the palladium catalyst/co-catalyst to form the compound of formula J-1, 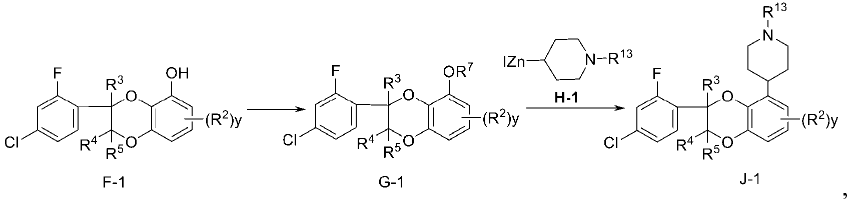 wherein R⁷, R¹³, R², R³, R⁴, R⁵, and y are as defined above.

In some embodiments, the compound of formula K is the compound of formula K-2. The method for preparing the compound of formula K-2 or a salt thereof further comprises the steps of converting the compound of formula F-2 to the compound of formula G-2, and reacting the compound of formula G-2 with a compound of formula H-1 in the presence of the palladium catalyst/co-catalyst to form the compound of formula J-2, 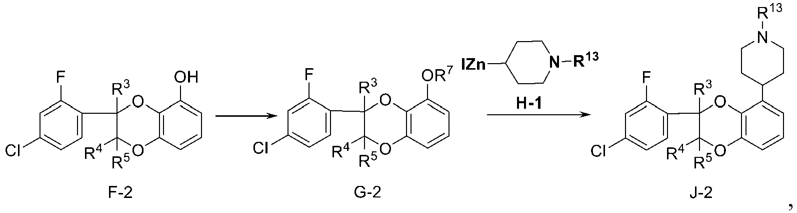 wherein R⁷, R¹³, R³, R⁴, and R⁵ are as defined above.

In some embodiments, the compound of formula K is the compound of formula K-3. The method for preparing the compound of formula K-3 or a salt thereof further comprises the steps of converting the compound of formula F-3 to the compound of formula G-3, and reacting the compound of formula G-3 with a compound of formula H-2 in the presence of the palladium catalyst/co-catalyst to form the compound of formula J-3, 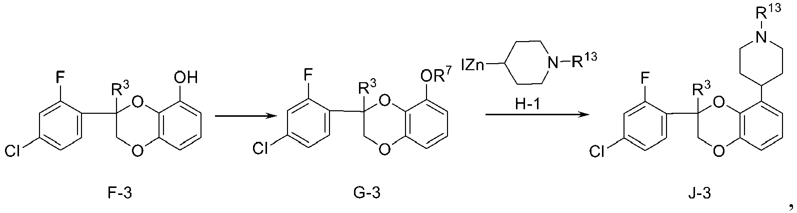 wherein R⁷, R¹³, and R³ are as defined above.

In some embodiments, the compound of formula K is the compound of formula K-4. The method for preparing the compound of formula K-4 or a salt thereof further comprises the steps of converting the compound of formula F-4 to the compound of formula G-4, and reacting the compound of formula G-4 with the compound of formula H-2 in the presence of Pd(dppf)Cl₂/cuprous iodide to form the compound of formula J-4,

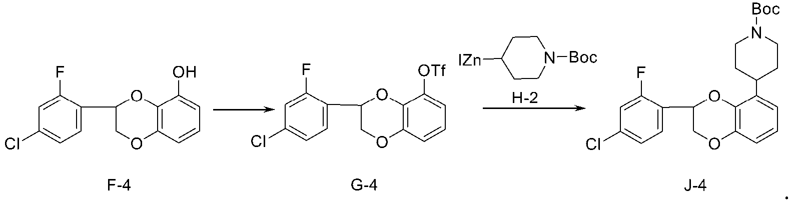

In some embodiments, the compound of formula K is the compound of formula K-4a. The method for preparing a compound of formula K-4a or a salt thereof further comprises the steps of converting the compound of formula F-4a to the compound of formula G-4a, and reacting the compound of formula G-4a with a compound of formula H-2 in the presence of Pd(dppf)Cl₂/cuprous iodide to form the compound of formula J-4a,

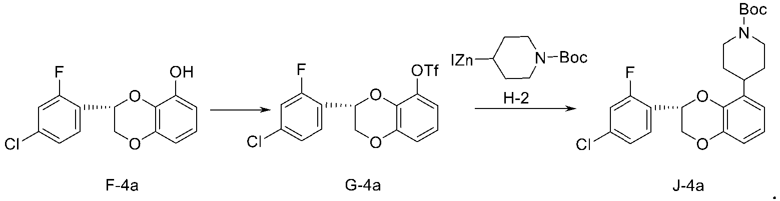

Further, the method of the present disclosure for preparing the compound of formula K or a salt thereof further comprises the step of converting the compound of formula J to the compound of formula K, 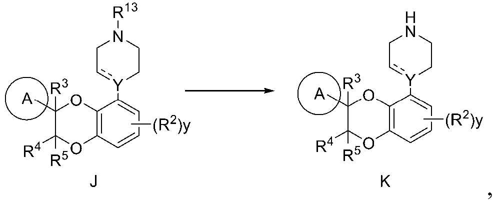 ring A, R², R³, R⁴, R⁵, y, R¹³, Y, -̅ -̅ -̅ are as defined above.

In some embodiments, further included is the step of converting the compound of formula J to the compound of formula K in the presence of hydrochloric acid, Me₃SiI, or p-toluenesulfonic acid. In some embodiments, further included is the step of converting the compound of formula J to the compound of formula K in the presence of hydrochloric acid.

In some embodiments, the solvent used for the reaction of the compound of formula J is selected from EtOAc, MeOH, CH₂Cl₂, CHCl₃, CH₃CN, or dioxane. In some embodiments, the solvent used for the reaction of the compound of formula J is selected from dioxane.

In some embodiments, the method for preparing the compound of formula K or a salt thereof further comprises a step of converting the compound of formula J to the compound of formula K in the presence of hydrochloric acid/dioxane.

In some embodiments, the compound of formula K is the compound of formula K-1. The method for preparing the compound of formula K-1 or a salt thereof further comprises a step of converting the compound of formula J-1 to the compound of formula K-1 in the presence of hydrochloric acid/dioxane, 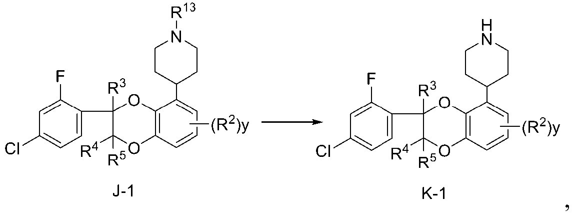 R², R³, R⁴, R⁵, y, R¹³ are as defined above.

In some embodiments, the compound of formula K is the compound of formula K-2. The method for preparing the compound of formula K-2 or a salt thereof further comprises a step of converting the compound of formula J-2 to the compound of formula K-2 in the presence of hydrochloric acid/dioxane, 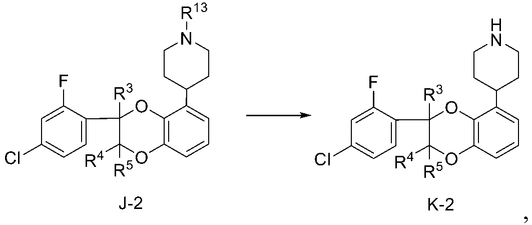 R³, R⁴, R⁵, and R¹³ are as defined above.

In some embodiments, the compound of formula K is the compound of formula K-3. The method for preparing the compound of formula K-3 or a salt thereof further comprises a step of converting the compound of formula J-3 to the compound of formula K-3 in the presence of hydrochloric acid/dioxane, 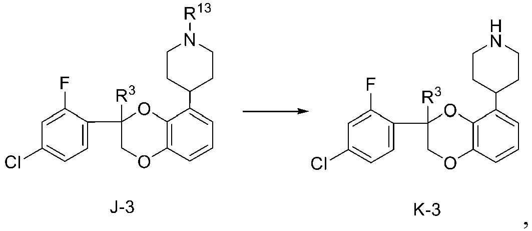 R³, and R¹³ are as defined above.

In some embodiments, the compound of formula K is the compound of formula K-4. The method for preparing the compound of formula K-4 or a salt thereof further comprises a step of converting the compound of formula J-4 to the compound of formula K-4 in the presence of hydrochloric acid/dioxane,

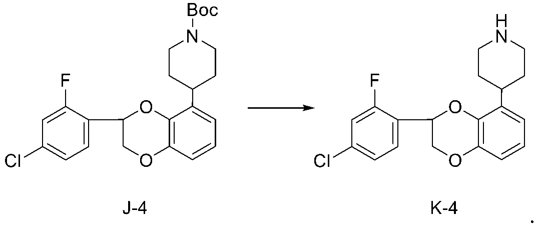

In some embodiments, the compound of formula K is the compound of formula K-4a. The method for preparing the compound of formula K-4a or a salt thereof further comprises a step of converting the compound of formula J-4a to the compound of formula K-4a in the presence of hydrochloric acid/dioxane,

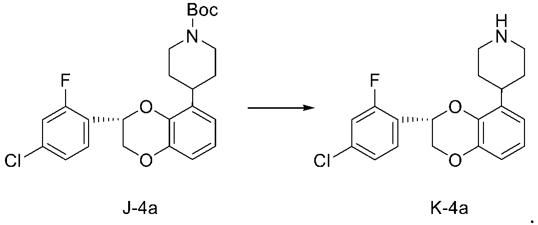

Some embodiments provide a method for preparing the compound of formula K or a salt thereof, comprising:
step 1) reacting the compound of formula (II) with the orthoester of formula (III) to form the compound of formula A, and reacting the compound of formula A with the compound of formula B in the presence of a base to form the compound of formula C;
step 2) forming the compound of formula D from the compound of formula C in the presence of the oxazaborolidine catalyst of formula (I)/a reducing agent;
step 3) converting the compound of formula D to the compound of formula E in the presence of an acid;
step 4) reacting the compound of formula E in the presence of trisubstituted phosphine/azodicarboxylate to form the compound of formula F;
step 5) converting the compound of formula F to the compound of formula G, and reacting the compound of formula G with the compound of formula H in the presence of palladium catalyst/co-catalyst to form the compound of formula J; and
Step 6) converting the compound of formula J to the compound of formula K; 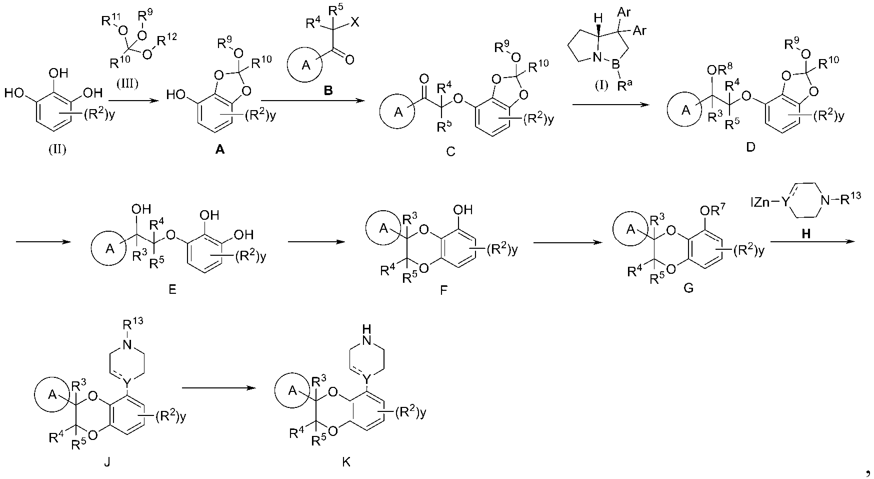 wherein, ring A, R², R³, R⁴, R⁵, R⁸, y, R⁹, R¹⁰, Ar, R^{a}, R¹¹, R¹², X, R⁷, and R¹³, -̅ -̅ -̅ are as defined above.

Some embodiments provide a method for preparing the compound of formula K-1 or a salt thereof, comprising:
step 1) reacting the compound of formula (II) with the orthoester of formula (III) to form the compound of formula A, and reacting the compound of formula A with the compound of formula B1 in the presence of a base to form the compound of formula C-1;
step 2) forming the compound of formula D-1 from the compound of formula C-1 in the presence of an oxazaborolidine catalyst of formula (I)/a reducing agent;
step 3) converting the compound of formula D-1 to the compound of formula E-1 in the presence of an acid;
step 4) reacting the compound of formula E-1 in the presence of trisubstituted phosphine/azodicarboxylate to form the compound of formula F-1;
step 5) converting the compound of formula F-1 to the compound of formula G-1, and reacting the compound of formula G-1 with the compound of formula H-1 in the presence of the palladium catalyst/co-catalyst to form the compound of formula J-1; and
step 6) converting the compound of formula J-1 to the compound of formula K-1 in the presence of hydrochloric acid/dioxane; 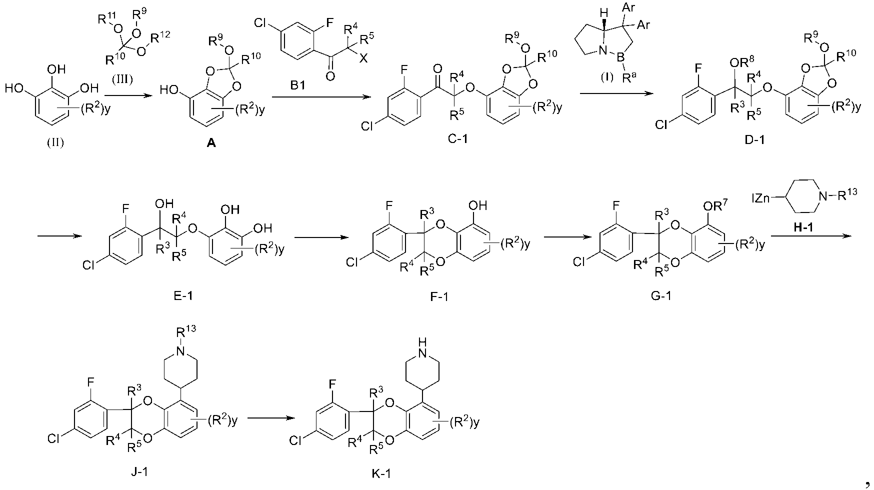 wherein, R², R³, R⁴, R⁵, R⁸, y, R⁹, R¹⁰, Ar, R^{a}, R¹¹, R¹², X, R⁷, and R¹³ are as defined above.

Some embodiments provide a method for preparing the compound of formula K-2 or a salt thereof, comprising:
step 1) reacting the compound of formula (IIa) with the orthoester of formula (III) to form the compound of formula A1, and reacting the compound of formula A1 with the compound of formula B1 in the presence of a base to form the compound of formula C-2;
step 2) forming the compound of formula D-2 from the compound of formula C-2 in the presence of an oxazaborolidine catalyst of formula (I)/a reducing agent;
step 3) converting the compound of formula D-2 to the compound of formula E-2 in the presence of an acid;
step 4) reacting the compound of formula E-2 in the presence of trisubstituted phosphine/azodicarboxylate to form the compound of formula F-2;
Step 5) converting the compound of formula F-2 to the compound of formula G-2, and reacting the compound of formula G-2 with the compound of formula H-1 in the presence of the palladium catalyst/co-catalyst to form the compound of formula J-2; and
step 6) converting the compound of formula J-2 to the compound of formula K-2 in the presence of hydrochloric acid/dioxane; 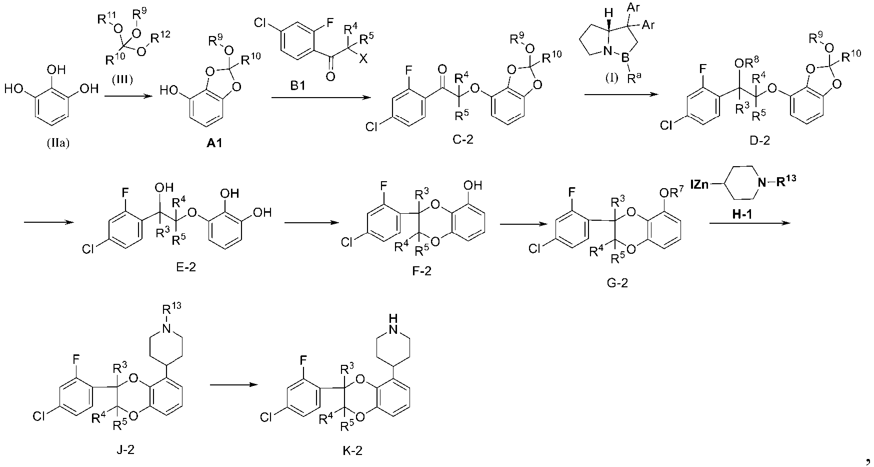 wherein, R³, R⁴, R⁵, R⁹, R¹⁰, Ar, R^{a}, R¹¹, R¹², X, R⁷, and R¹³ are as defined above.

Some embodiments provide a method for preparing the compound of formula K-3 or a salt thereof, comprising:
step 1) reacting the compound of formula (IIa) with the orthoester of formula (III) to form the compound of formula A1, and reacting the compound of formula A1 with the compound of formula B2 in the presence of a base to form the compound of formula C-3;
step 2) forming the compound of formula D-3 from the compound of formula C-3 in the presence of an oxazaborolidine catalyst of formula (I)/a reducing agent;
step 3) converting the compound of formula D-3 to the compound of formula E-3 in the presence of an acid;
step 4) reacting the compound of formula E-3 in the presence of trisubstituted phosphine/azodicarboxylate to form the compound of formula F-3;
Step 5) converting the compound of formula F-3 to the compound of formula G-3, and reacting the compound of formula G-3 with the compound of formula H-2 in the presence of the palladium catalyst/co-catalyst to form the compound of formula J-3; and
step 6) converting the compound of formula J-3 to the compound of formula K-3 in the presence of hydrochloric acid/dioxane; 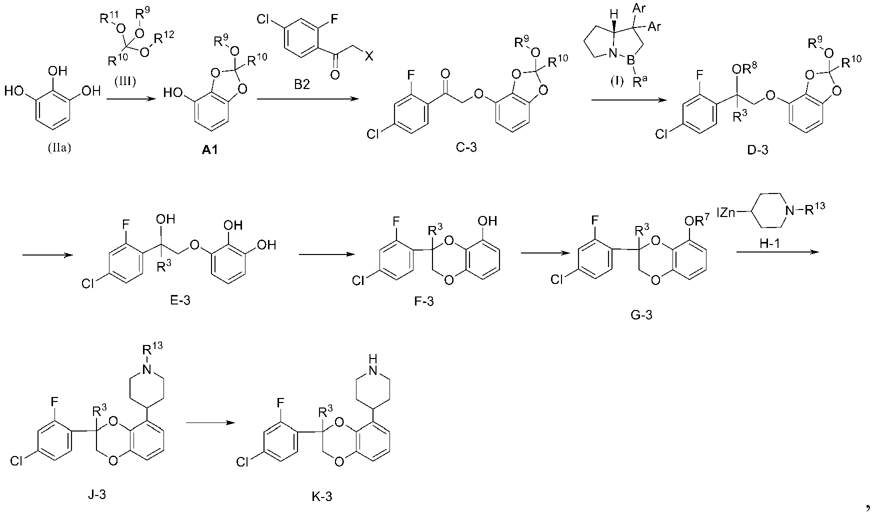 wherein R⁷, R¹³, X, Ar, R^{a}, R⁸, R⁹, R¹⁰, R³, R¹¹, and R¹² are as defined above.

Some embodiments provide a method for preparing a compound of formula K-4 or a salt thereof, comprising:
step 1) reacting a compound of formula (IIa) with an orthoester of formula (IIIa) to form a compound of formula A2, and reacting the compound of formula A2 with a compound of formula B3 in the presence of potassium carbonate to form a compound of formula C-4;
step 2) forming the compound of formula D-4a from the compound of formula C-4 in the presence of the oxazaborolidine catalyst of formula (I)/a reducing agent;
step 3) converting the compound of formula D-4 to the compound of formula E-4 in the presence of hydrochloric acid/methanol;
step 4) reacting the compound of formula E-4 in the presence of triphenylphosphine/diisopropyl azodicarboxylate to form a compound of formula F-4;
step 5) converting the compound of formula F-4 to the compound of formula G-4, and reacting the compound of formula G-4 with the compound of formula H-2 in the presence of Pd(dppf)Cl₂/cuprous iodide to form a compound of formula J-4; and
step 6) converting the compound of formula J-4 to the compound of formula K-4 in the presence of hydrochloric acid/dioxane; 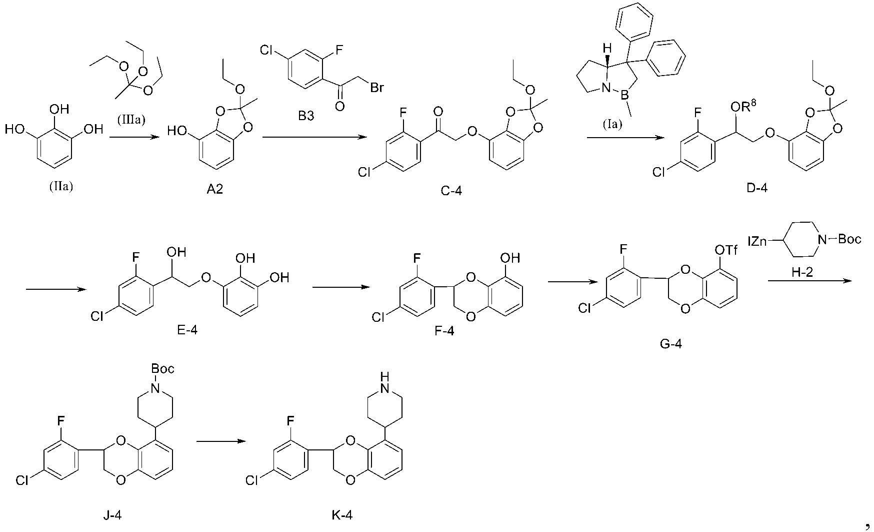 wherein, R⁸ is as defined above.

Some embodiments provide a method for preparing the compound of formula K-4a or a salt thereof, comprising:
step 1) reacting a compound of formula (IIa) with an orthoester of formula (IIIa) to form a compound of formula A2, and reacting the compound of formula A2 with a compound of formula B3 in the presence of potassium carbonate to form a compound of formula C-4;
step 2) forming a compound of formula D-4a from the compound of C-4 in the presence of the oxazaborolidine catalyst of formula (Ia)/BH₃·THF;
step 3) reacting the compound of formula D-4a in the presence of hydrochloric acid/methanol to form a compound of formula E-4a
step 4) a step of reacting the compound of formula E-4a in the presence of triphenylphosphine/diisopropyl azodicarboxylate to form a compound of formula F-4a,
step 5) converting the compound of formula F-4a to the compound of formula G-4a, and reacting the compound of formula G-4a with a compound of formula H-2 in the presence of Pd(dppf)Cl₂/cuprous iodide to form a compound of formula J-4a; and
step 6) converting the compound of formula J-4a to the compound of formula K-4a in the presence of hydrochloric acid/dioxane; 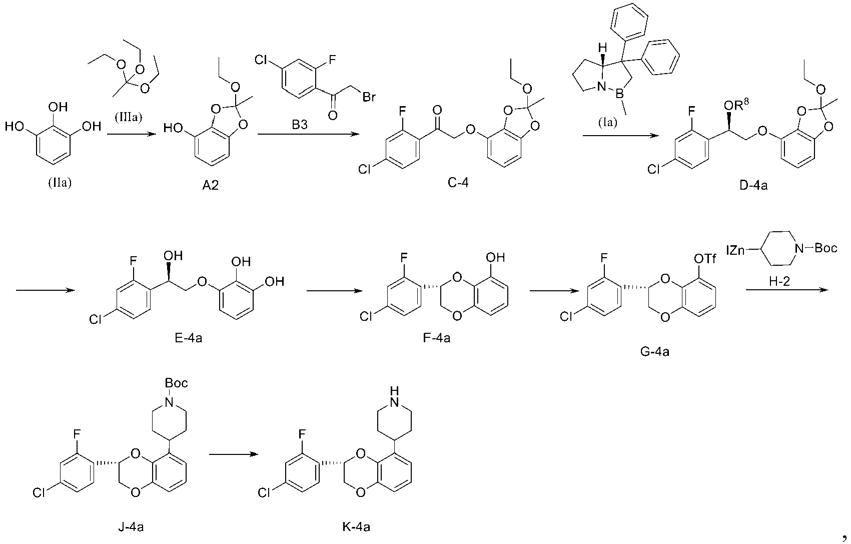 wherein, R⁸ is as defined above.

The present disclosure provides a method for preparing the compound of formula F or a salt thereof, 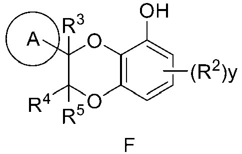 the method comprises a step of reacting a compound of formula E in the presence of trisubstituted phosphine/azodicarboxylate to form a compound of formula F, 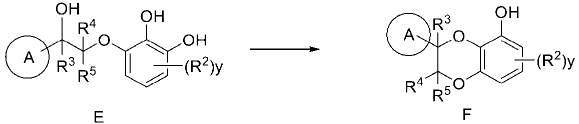 wherein,
ring A is selected from 6- to 10-membered aryl or 5- to 10-membered heteroaryl, wherein the aryl or heteroaryl is optionally substituted with one or more halogen, cyano, C₁₋₆ alkyl or C₁₋₆ alkoxy;
"-̅ -̅ -̅" is a double bond or a single bond,
when "-̅ -̅ -̅" is a single bond, Y is selected from N or CR¹⁴,
when "-̅ -̅ -̅" is a double bond, Y is CR¹⁴;
R² is selected from halogen, cyano, C₁₋₆ alkyl or C₁₋₆ alkoxy;
R³, R⁴, and R⁵ are each independently selected from hydrogen, halogen, cyano, C₁₋₆ alkyl or C₁₋₆ alkoxy, wherein the alkyl or alkoxy is optionally substituted with one or more halogen, hydroxyl, cyano, or amino;
R¹⁴ is selected from hydrogen, halogen, cyano, C₁₋₆ alkyl or C₁₋₆ alkoxy, wherein the alkyl or alkoxy is optionally substituted with one or more halogen, hydroxyl, cyano, or amino;
y is selected from 0, 1, 2, or 3.

In some embodiments, the trisubstituted phosphine is triphenylphosphine.

In some embodiments, the azodicarboxylate is selected from diethyl azodicarboxylate, diisopropyl azodicarboxylate, or di(4-chlorobenzyl) azodicarboxylate. In certain embodiments, the azodicarboxylate is diisopropyl azodicarboxylate.

In some embodiments, the reaction solvent for the compound of formula E is selected from tetrahydrofuran, diethyl ether, dichloromethane, toluene, ethyl acetate, acetonitrile, and N,N-dimethylformamide. In certain embodiments, the reaction solvent for the compound of formula E is dichloromethane.

In some embodiments, the molar ratio of the compound of formula E to the trisubstituted phosphine is 1:1 to 1:2, including but not limited to 1:1, 1:1.1, 1:1.2, 1:1.3, 1:1.4, 1:1.5, 1:1.6, 1:1.7, 1:1.8, 1:1.9, 1:2 or any value between any two numbers. In certain embodiments, the molar ratio of the compound of formula E to the trisubstituted phosphine is 1:1 to 1:1.5.

In certain embodiments, the molar ratio of the compound of formula E to the triphenylphosphine is 1:1 to 1:1.5.

In some embodiments, the molar ratio of the compound of formula E to the azodicarboxylate is 1:1 to 1:2, including but not limited to 1:1, 1:1.1, 1:1.2, 1:1.3, 1:1.4, 1:1.5, 1:1.6, 1:1.7, 1:1.8, 1:1.9, 1:2, or any value between any two numbers. In certain embodiments, the molar ratio of the compound of formula E to the azodicarboxylate is 1:1 to 1:1.5.

In certain embodiments, the molar ratio of the compound of formula E to the diisopropyl azodicarboxylate is 1:1 to 1:1.5.

In some embodiments, ring A is selected from 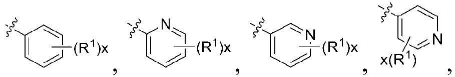 or 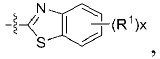 R¹ is selected from fluoro, chloro, bromo, iodo, or cyano, and x is selected from 0, 1, 2, 3, 4.

In certain embodiments, ring A is selected from 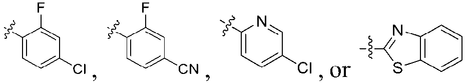 . In certain embodiments, ring A is

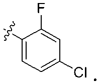

In some embodiments, the compound of formula F is the compound of formula F-1, 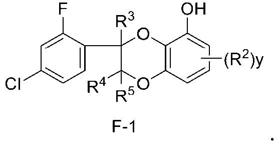 The method for preparing the compound of formula F-1 or a salt thereof comprises the step of reacting the compound of formula E-1 in the presence of trisubstituted phosphine/azodicarboxylate to form the compound of formula F-1, 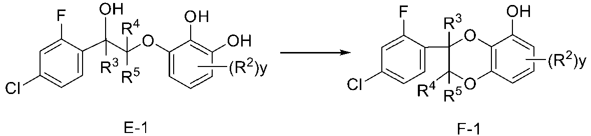 wherein R², R³, R⁴, R⁵, R⁶, R⁸, and y are as defined above.

In some embodiments, y is 0.

In some embodiments, the compound of formula F is the compound of formula F-2, 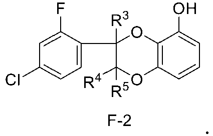 The method for preparing the compound of formula F-2 or a salt thereof comprises the step of reacting the compound of formula E-2 in the presence of trisubstituted phosphine/azodicarboxylate to form the compound of formula F-2, 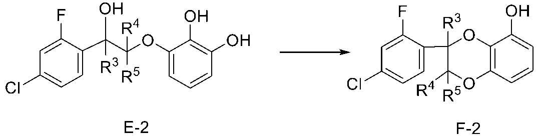 wherein R³, R⁴, and R⁵ are as defined above.

In some embodiments, R⁴ and R⁵ are each independently selected from hydrogen, methyl, ethyl, propyl, butyl, fluoro, chloro, bromo, and cyano. In certain specific embodiments, R⁴ and R⁵ are each independently hydrogen.

In some embodiments, the compound of formula F is the compound of formula F-3, 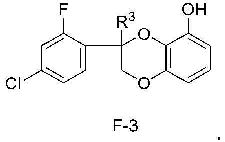 The method for preparing the compound of formula F-3 or a salt thereof comprises the step of reacting the compound of formula E-3 in the presence of trisubstituted phosphine/azodicarboxylate to form the compound of formula F-3, 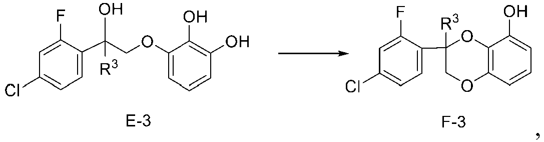 wherein, R³ is as defined above.

In some embodiments, R³ is selected from hydrogen, methyl, ethyl, propyl, butyl, fluoro, chloro, bromo, and cyano. In certain specific embodiments, R³ is selected from hydrogen and methyl.

In some embodiments, the compound of formula F is the compound of formula F-4, 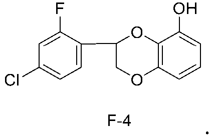 The method for preparing the compound of formula F-4 or a salt thereof comprises a step of reacting a compound of formula E-4 in the presence of triphenylphosphine/diisopropyl azodicarboxylate to form a compound of formula F-4,

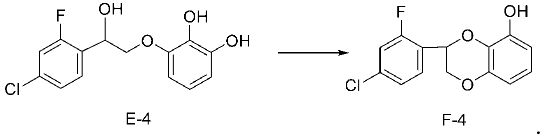

In some embodiments, the compound of formula F is the compound of formula F-4a, 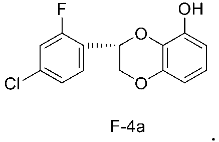 The method for preparing the compound of formula F-4a or a salt thereof comprises a step of reacting a compound of formula E-4a in the presence of triphenylphosphine/diisopropyl azodicarboxylate to form the compound of formula F-4a,

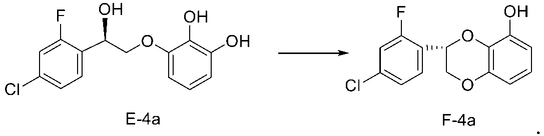

Further, the method of the present disclosure for preparing the compound of formula F or a salt thereof further comprises a step of converting the compound of formula D to a compound of formula M in the presence of an acid, and a step of converting the compound of formula M to the compound of formula E, 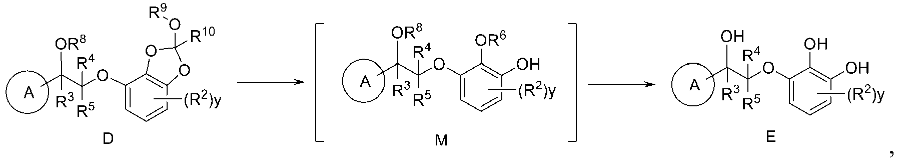 wherein,
R⁶ and R⁸ are each independently selected from hydrogen or a hydroxyl protecting group;
R⁹ and R¹⁰ are each independently selected from hydrogen, C₁₋₆ alkyl or C₁₋₆ alkoxy, wherein the alkyl or alkoxy is optionally substituted with one or more halogen, hydroxyl, cyano, or amino;
Ring A, R², R³, R⁴, R⁵, and y are as defined above.

In some embodiments, the acid is selected from hydrochloric acid, sulfuric acid, formic acid, acetic acid, and p-toluenesulfonic acid. In certain embodiments, the acid is hydrochloric acid.

In some embodiments, the reaction solvent for the compound of formula D is an alcohol. In some embodiments, the alcohol is selected from methanol, ethanol, propanol, butanol, pentanol, and hexanol. In certain embodiments, the alcohol is methanol.

In some embodiments, the compound of formula F is the compound of formula F-1. The method for preparing the compound of formula F-1 or a salt thereof further comprises a step of converting the compound of formula D-1 to the compound of formula M-1 in the presence of an acid, and a step of converting the compound of formula M-1 to the compound of formula E-1, 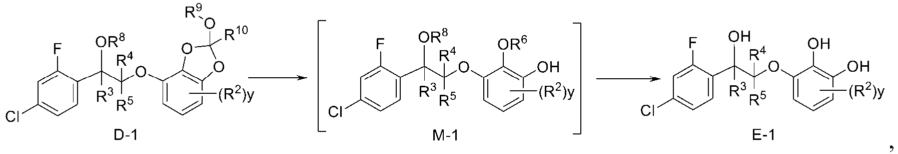 wherein, R⁹, R¹⁰, R², R³, R⁴, R⁵, R⁶, R⁸, and y are as defined above.

In some embodiments, the compound of formula F is the compound of formula F-2. The method for preparing the compound of formula F-2 or a salt thereof further comprises a step of converting the compound of formula D-2 to the compound of formula M-2 in the presence of an acid, and a step of converting the compound of formula M-2 to the compound of formula E-2, 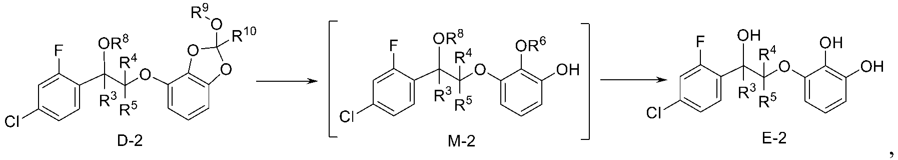 wherein, R⁶, R⁸, R⁹, R¹⁰, R³, R⁴, and R⁵ are as defined above.

In some embodiments, the compound of formula F is the compound of formula F-3. The method for preparing the compound of formula F-3 or a salt thereof further comprises a step of converting the compound of formula D-3 to the compound of formula M-3 in the presence of an acid, and a step of converting the compound of formula M-3 to the compound of formula E-3, 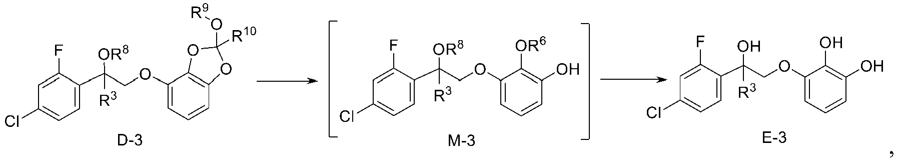 wherein, R⁶, R⁸, R⁹, R¹⁰, and R³ are as defined above.

In some embodiments, R⁹ is selected from hydrogen, methyl, ethyl, propyl, butyl, and isopropyl. In certain embodiments, R⁹ is ethyl.

In some embodiments, R¹⁰ is selected from hydrogen, methyl, ethyl, propyl, butyl, and isopropyl. In certain embodiments, R¹⁰ is methyl.

In some embodiments, the compound of formula F is the compound of formula F-4. The method for preparing the compound of formula F-4 or a salt thereof further comprises a step of converting the compound of formula D-4 to the compound of formula M-4 in the presence of hydrochloric acid/methanol, and a step of converting the compound of formula M-4 to the compound of formula E-4, 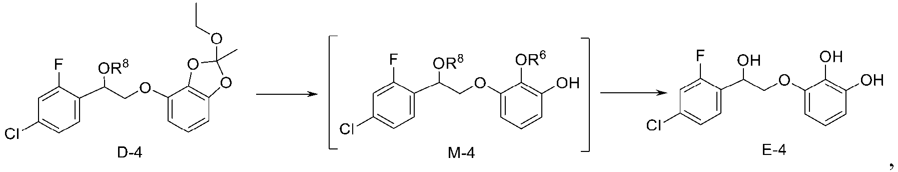 wherein, R⁶ and R⁸ are as defined above.

In some embodiments, the compound of formula F is the compound of formula F-4a. The method for preparing the compound of formula F-4a or a salt thereof further comprises a step of converting the compound of formula D-4a to a compound of formula M-4a in the presence of hydrochloric acid/methanol, and comprises a step of converting the compound of formula M-4a to the compound of formula E-4a, 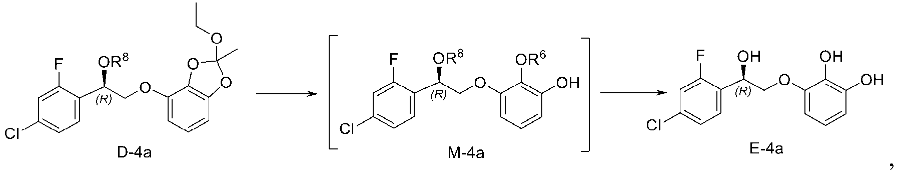 wherein, R⁶ and R⁸ are as defined above.

Further, the method of the present disclosure for preparing the compound of formula F or a salt thereof further comprises a step of forming the compound of formula D from the compound of formula C in the presence of an oxazaborolidine catalyst of formula (I)/a reducing agent, 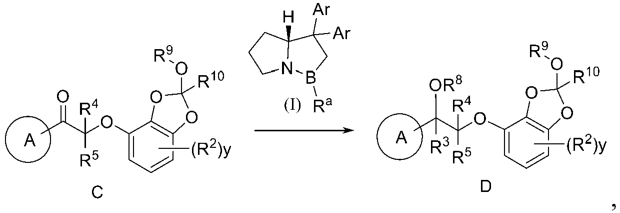
wherein: each Ar is independently selected from 6-membered aryl or 5- to 10-membered heteroaryl, wherein the aryl or heteroaryl is optionally substituted with one or more halogen, cyano, C₁₋₆ alkyl, or C₁₋₆ alkoxy;
R^{a} is selected from hydrogen or C₁₋₆ alkyl, wherein the alkyl is optionally substituted with one or more halogen, hydroxyl, cyano, or amino;
ring A, R⁸, R⁹, R¹⁰, R², R³, R⁴, R⁵, and y are as defined above.

In certain embodiments, each Ar is independently phenyl.

In certain embodiments, R^{a} is C₁₋₆ alkyl. In certain embodiments, R^{a} is methyl, ethyl, n-propyl, or isopropyl. In certain embodiments, R^{a} is methyl.

In certain embodiments, the oxazaborolidine catalyst of formula (I) is represented by formula (Ia)

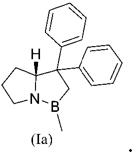

In some embodiments, the reducing agent is a borane-ligand compound. In certain specific embodiments, the reducing agent is BH₃·THF, BH₃·Me₂S, or BH₃·DEA. In certain specific embodiments, the reducing agent is BH₃·THF.

In some embodiments, the compound of formula F is the compound of formula F-1. The method for preparing the compound of formula F-1 or a salt thereof further comprises a step of forming the compound of formula D-1 from the compound of formula C-1 in the presence of an oxazaborolidine catalyst of formula (I)/a reducing agent, 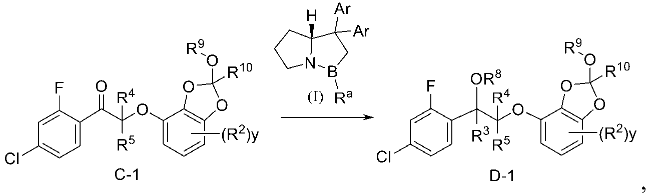 wherein Ar, R^{a}, R⁸, R⁹, R¹⁰, R², R³, R⁴, R⁵, and y are as defined above.

In some embodiments, the compound of formula F is the compound of formula F-2. The method for preparing the compound of formula F-2 or a salt thereof further comprises a step of forming the compound of formula D-2 from the compound of formula C-2 in the presence of an oxazaborolidine catalyst of formula (I)/a reducing agent, 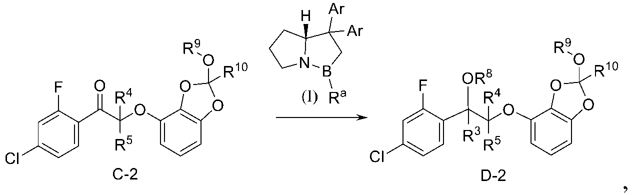 wherein Ar, R^{a}, R⁸, R⁹, R¹⁰, R², R³, R⁴, R⁵, and y are as defined above.

In some embodiments, the compound of formula F is the compound of formula F-3. The method for preparing the compound of formula F-3 or a salt thereof further comprises a step of forming the compound of formula D-3 from the compound of formula C-3 in the presence of an oxazaborolidine catalyst of formula (I)/a reducing agent, 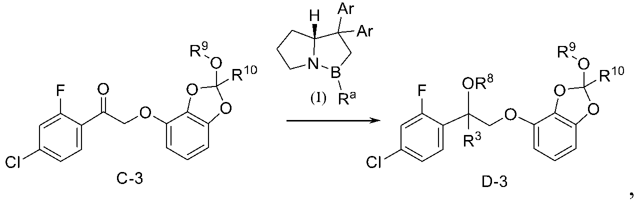 wherein Ar, R^{a}, R⁸, R⁹, R¹⁰, and R³ are as defined above.

In some embodiments, the compound of formula F is the compound of formula F-4. The method for preparing the compound of formula F-4 or a salt thereof further comprises a step of forming the compound of formula D-4 from the compound of formula C-4 in the presence of an oxazaborolidine catalyst of formula (Ia)/a reducing agent, 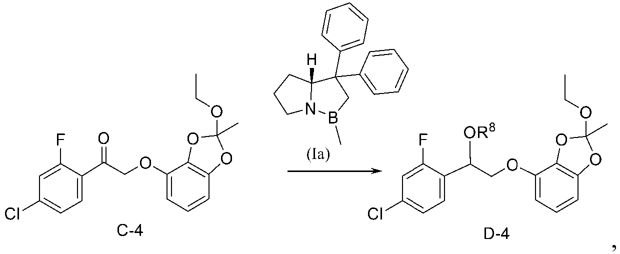 wherein R⁸ is as defined above.

In some embodiments, the compound of formula F is the compound of formula F-4a. The method for preparing the compound of formula F-4a or a salt thereof further comprises a step of forming the compound of formula D-4a from the compound of formula C-4 in the presence of an oxazaborolidine catalyst of formula (Ia)/BH₃·THF,

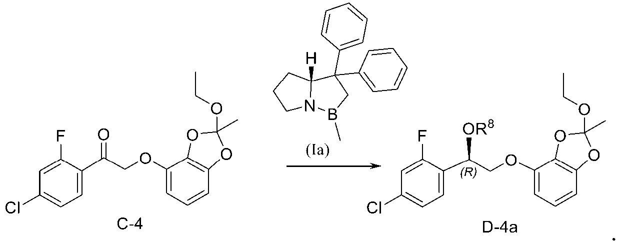

Further, the method of the present disclosure for preparing the compound of formula F or a salt thereof further comprises steps of reacting the compound of formula (II) with the orthoester of formula (III) to form the compound of formula A, and reacting the compound of formula A with the compound of formula B in the presence of a base to form the compound of formula C, 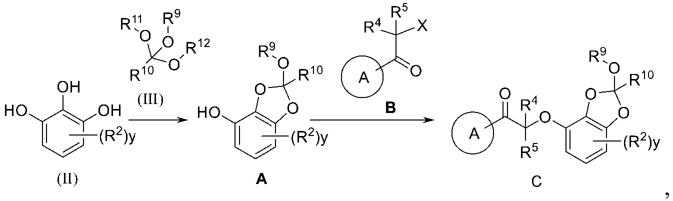
wherein: R¹¹ and R¹² are each independently selected from hydrogen, C₁₋₆ alkyl, or C₁₋₆ alkoxy, wherein the alkyl or alkoxy is optionally substituted with one or more halogen, hydroxyl, cyano, or amino;
X is halogen;
ring A, R⁹, R¹⁰, R², R⁴, R⁵, and y are as defined above.

In some embodiments, the base is selected from potassium carbonate and sodium carbonate. In certain embodiments, the base is potassium carbonate.

In some embodiments, the compound of formula F is the compound of formula F-1. The method for preparing the compound of formula F-1 or a salt thereof further comprises steps of reacting the compound of formula (II) with the orthoester of formula (III) to form the compound of formula A, and reacting the compound of formula A with the compound of formula B1 in the presence of a base to form the compound of formula C-1, 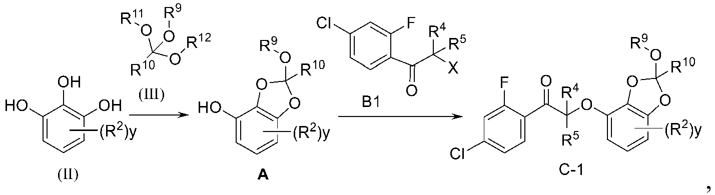 wherein: R⁹, R¹⁰, R¹¹, R¹², X, R², R⁴, R⁵, and y are as defined above.

In some embodiments, the compound of formula F is the compound of formula F-2. The method for preparing the compound of formula F-2 or a salt thereof further comprises steps of reacting the compound of formula (IIa) with the orthoester of formula (III) to form the compound of formula A1, and reacting the compound of formula A1 with the compound of formula B1 in the presence of a base to form the compound of formula C-2, 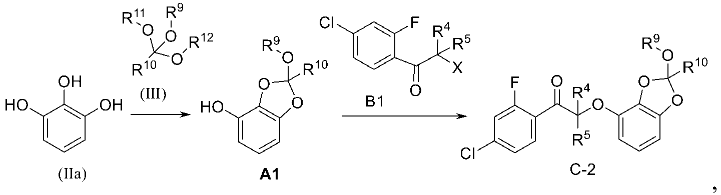 wherein: R⁹, R¹⁰, R¹¹, R¹², X, R⁴, and R⁵ are as defined above.

In some embodiments, the compound of formula F is the compound of formula F-3. The method for preparing the compound of formula F-3 or a salt thereof further comprises steps of reacting the compound of formula (IIa) with the orthoester of formula (III) to form the compound of formula A1, and reacting the compound of formula A1 with the compound of formula B2 in the presence of a base to form the compound of formula C-3, 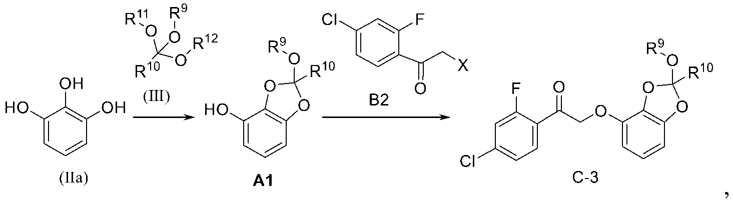 wherein: R⁹, R¹⁰, R¹¹, R¹², and X are as defined above.

In some embodiments, X is selected from fluoro, chloro, bromo, and iodo. In certain specific embodiments, X is bromo.

In some embodiments, R¹¹ and R¹² are each independently C₁₋₆ alkyl. In some embodiments, R¹¹ and R¹² are each independently selected from methyl, ethyl, propyl, butyl, and isopropyl. In certain specific embodiments, R¹¹ and R¹² are both ethyl.

In some embodiments, the compound of formula F is the compound of formula F-4. The method for preparing the compound of formula F-4 or a salt thereof further comprises steps of reacting the compound of formula (IIa) with an orthoester of formula (IIIa) to form the compound of formula A2, and reacting the compound of formula A2 with the compound of formula B3 in the presence of potassium carbonate to form the compound of formula C-4,

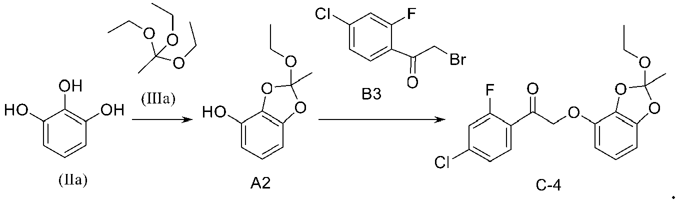

Some embodiments provide a method for preparing the compound of formula F or a salt thereof, comprising:
step 1) reacting the compound of formula (II) with the orthoester of formula (III) to form the compound of formula A, and reacting the compound of formula A with the compound of formula B in the presence of a base to form the compound of formula C;
step 2) forming the compound of formula D from the compound of formula C in the presence of the oxazaborolidine catalyst of formula (I)/a reducing agent;
step 3) converting the compound of formula D to the compound of formula E in the presence of an acid; and
step 4) reacting the compound of formula E in the presence of trisubstituted phosphine/azodicarboxylate to form the compound of formula F; 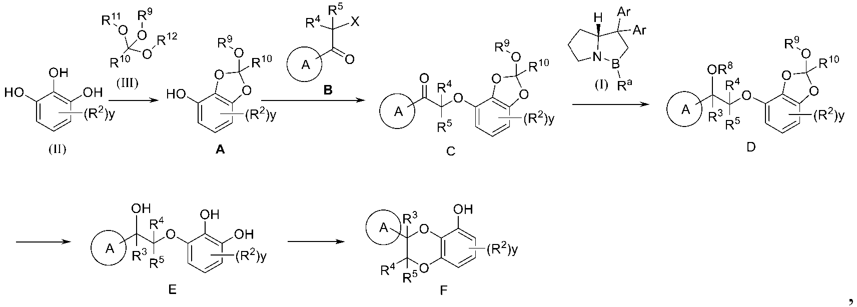 wherein ring A, R², R³, R⁴, R⁵, R⁸, y, R⁹, R¹⁰, Ar, R^{a}, R¹¹, R¹², and X are as defined above.

Some embodiments provide a method for preparing the compound of formula F-1 or a salt thereof, comprising:
step 1) reacting the compound of formula (II) with the orthoester of formula (III) to form the compound of formula A, and reacting the compound of formula A with the compound of formula B1 in the presence of a base to form the compound of formula C-1;
step 2) forming the compound of formula D-1 from the compound of formula C-1 in the presence of an oxazaborolidine catalyst of formula (I)/a reducing agent;
step 3) converting the compound of formula D-1 to the compound of formula E-1 in the presence of an acid; and
step 4) reacting the compound of formula E-1 in the presence of trisubstituted phosphine/azodicarboxylate to form the compound of formula F-1; 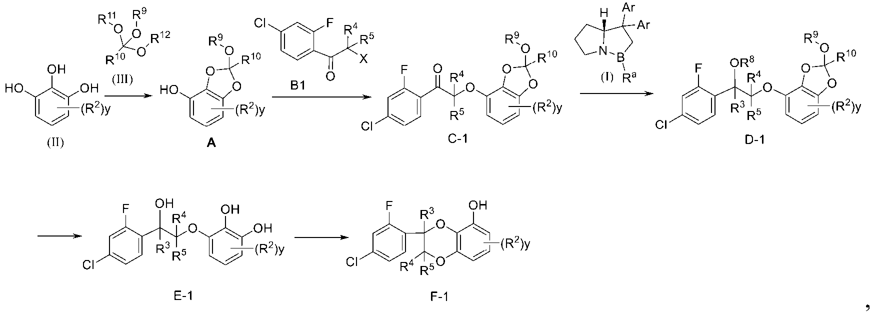 wherein, R², R³, R⁴, R⁵, R⁸, y, R⁹, R¹⁰, Ar, R^{a}, R¹¹, R¹², and X are as defined above.

Some embodiments provide a method for preparing the compound of formula F-2 or a salt thereof, comprising:
step 1) reacting the compound of formula (IIa) with the orthoester of formula (III) to form the compound of formula A1, and reacting the compound of formula A1 with the compound of formula B1 in the presence of a base to form the compound of formula C-2;
step 2) forming the compound of formula D-2 from the compound of formula C-2 in the presence of an oxazaborolidine catalyst of formula (I)/a reducing agent;
step 3) converting the compound of formula D-2 to the compound of formula E-2 in the presence of an acid; and
step 4) reacting the compound of formula E-2 in the presence of trisubstituted phosphine/azodicarboxylate to form the compound of formula F-2; 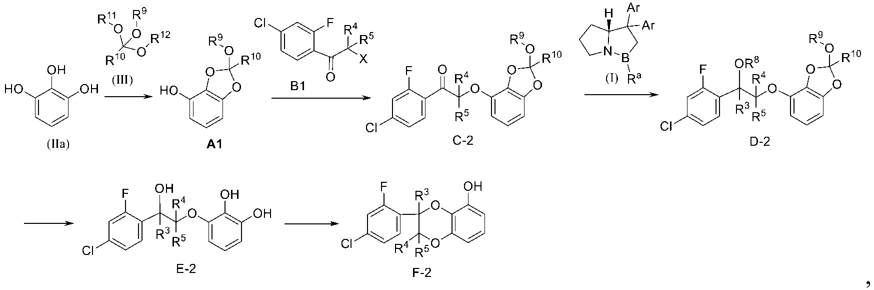 wherein R³, R⁴, R⁵, R⁹, R¹⁰, Ar, R^{a}, R¹¹, R¹², and X are as defined above.

Some embodiments provide a method for preparing the compound of formula F-3 or a salt thereof, comprising:
step 1) reacting the compound of formula (IIa) with the orthoester of formula (III) to form the compound of formula A1, and reacting the compound of formula A1 with the compound of formula B2 in the presence of a base to form the compound of formula C-3;
step 2) forming the compound of formula D-3 from the compound of formula C-3 in the presence of an oxazaborolidine catalyst of formula (I)/a reducing agent;
step 3) converting the compound of formula D-3 to the compound of formula E-3 in the presence of an acid; and
step 4) reacting the compound of formula E-3 in the presence of trisubstituted phosphine/azodicarboxylate to form the compound of formula F-3; 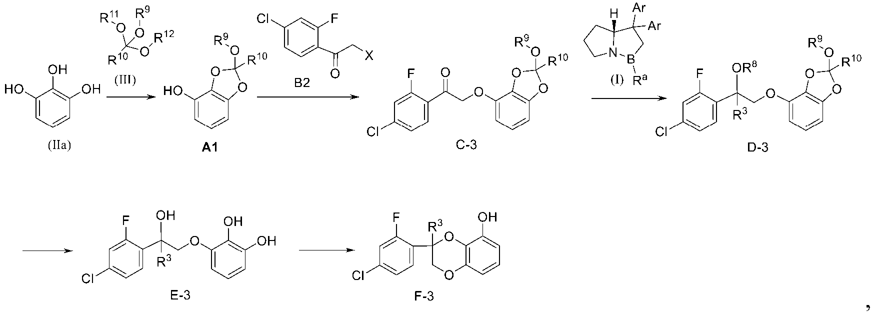 wherein X, Ar, R^{a}, R⁸, R⁹, R¹⁰, R³, R¹¹, and R¹² are as defined above.

Some embodiments provide a method for preparing the compound of formula F-4 or a salt thereof, comprising:
step 1) reacting a compound of formula (IIa) with an orthoester of formula (IIIa) to form a compound of formula A2, and reacting the compound of formula A2 with a compound of formula B3 in the presence of potassium carbonate to form a compound of formula C-4;
step 2) forming the compound of formula D-4a from the compound of formula C-4 in the presence of the oxazaborolidine catalyst of formula (I)/a reducing agent;
step 3) converting the compound of formula D-4 to the compound of formula E-4 in the presence of hydrochloric acid/methanol; and
step 4) reacting the compound of formula E-4 in the presence of triphenylphosphine/diisopropyl azodicarboxylate to form a compound of formula F-4; 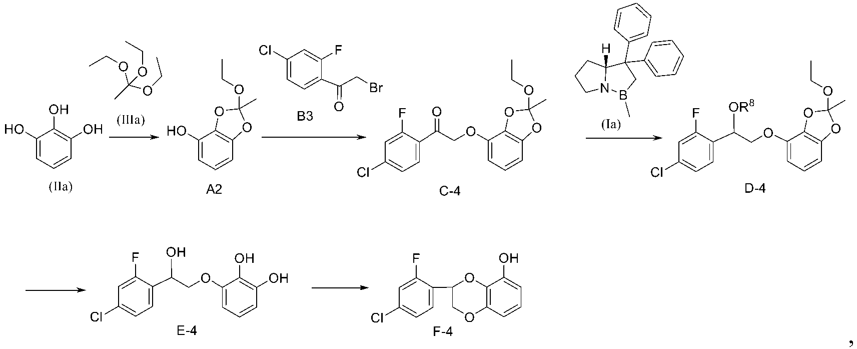 wherein, R⁸ is as defined above.

Some embodiments provide a method for preparing the compound of formula F-4a or a salt thereof, comprising:
step 1) reacting a compound of formula (IIa) with an orthoester of formula (IIIa) to form a compound of formula A2, and reacting the compound of formula A2 with a compound of formula B3 in the presence of potassium carbonate to form a compound of formula C-4;
step 2) forming a compound of formula D-4a from the compound of C-4 in the presence of the oxazaborolidine catalyst of formula (Ia)/BH₃·THF;
step 3) reacting the compound of formula D-4a in the presence of hydrochloric acid/methanol to form a compound of formula E-4a and
step 4) a step of reacting the compound of formula E-4a in the presence of triphenylphosphine/diisopropyl azodicarboxylate to form a compound of formula F-4a, 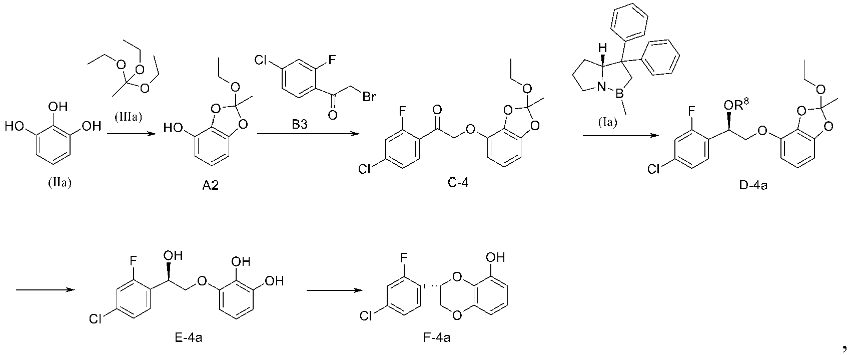 wherein, R⁸ is as defined above.

In another aspect, the present disclosure further provides the compound of formula C or a salt thereof, 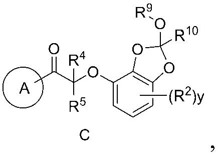 wherein ring A, R², y, R⁴, R⁵, R⁹, R¹⁰ are as defined above.

In some embodiments, the compound of formula C or a salt thereof is the compound of formula C-1 or a salt thereof, 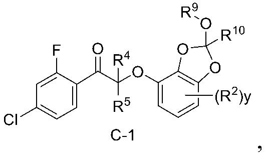 wherein R², y, R⁴, R⁵, R⁹, R¹⁰ are as defined above.

In some embodiments, the compound of formula C or a salt thereof is the compound of formula C-2 or a salt thereof, 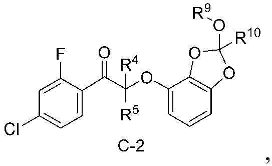 wherein R⁴, R⁵, R⁹, and R¹⁰ are as defined above.

In some embodiments, the compound of formula C or a salt thereof is the compound of formula C-3 or a salt thereof, 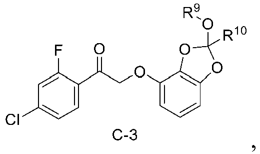 wherein R⁹ and R¹⁰ are as defined above.

In some embodiments, the compound of formula C or a salt thereof is the compound of formula C-4 or a salt thereof,

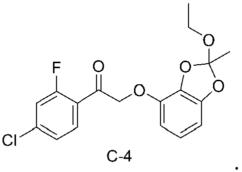

In another aspect, the present disclosure also provides the compound of formula D or a salt thereof, 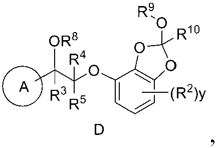 wherein ring A, R², y, R³, R⁴, R⁵, R⁸, R⁹, and R¹⁰ are as defined above.

In some embodiments, the compound of formula D or a salt thereof is the compound of formula D-1 or a salt thereof, 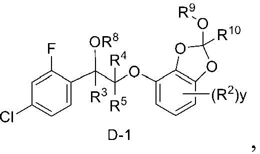 wherein R², y, R³, R⁴, R⁵, R⁸, R⁹, and R¹⁰ are as defined above.

In some embodiments, the compound of formula D or a salt thereof is the compound of formula D-2 or a salt thereof, 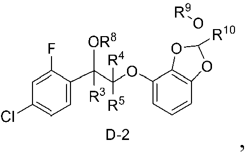 wherein R³, R⁴, R⁵, R⁸, R⁹, and R¹⁰ are as defined above.

In some embodiments, the compound of formula D or a salt thereof is the compound of formula D-3 or a salt thereof, 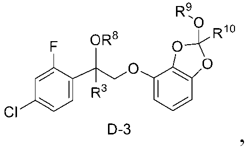 wherein R³, R⁸, R⁹, and R¹⁰ are as defined above.

In some embodiments, the compound of formula D or a salt thereof is the compound of formula D-4 or a salt thereof, 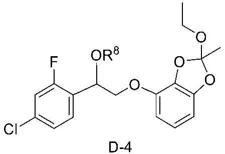 wherein R⁸ is as defined above.

In some embodiments, the compound of formula D or a salt thereof is the compound of formula D-4a or a salt thereof, 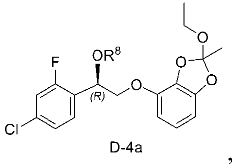 wherein R⁸ is as defined above.

In another aspect, the present disclosure also provides the compound of formula E or a salt thereof, 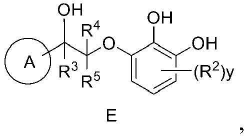 wherein ring A, R², y, R³, R⁴, and R⁵ are as defined above.

In some embodiments, the compound of formula E or a salt thereof is the compound of formula E-1 or a salt thereof, 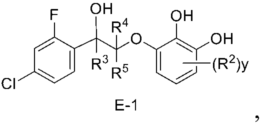 wherein R², y, R³, R⁴, and R⁵ are as defined above.

In some embodiments, the compound of formula E or a salt thereof is the compound of formula E-2 or a salt thereof, 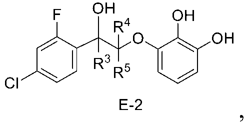 wherein R³, R⁴, and R⁵ are as defined above.

In some embodiments, the compound of formula E or a salt thereof is the compound of formula E-3 or a salt thereof, 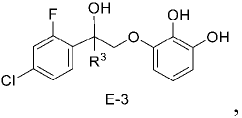 wherein R³ is as defined above.

In some embodiments, the compound of formula E or a salt thereof is the compound of formula E-4 or a salt thereof,

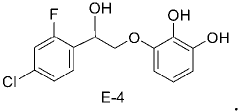

In some embodiments, the compound of formula E or a salt thereof is the compound of formula E-4a or a salt thereof,

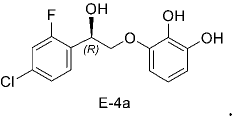

In another aspect, the present disclosure further provides the compound of formula M or a salt thereof, 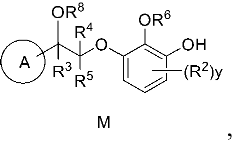 wherein ring A, R², y, R³, R⁴, R⁵, R⁶, R⁸ are as defined above.

In some embodiments, the compound of formula M or a salt thereof is the compound of formula M-1 or a salt thereof, 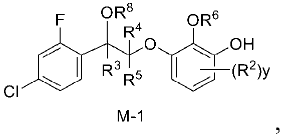 wherein R², y, R³, R⁴, R⁵, R⁶, and R⁸ are as defined above.

In some embodiments, the compound of formula M or a salt thereof is the compound of formula M-2 or a salt thereof, 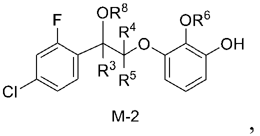 wherein R³, R⁴, R⁵, R⁶, and R⁸ are as defined above.

In some embodiments, the compound of formula M or a salt thereof is the compound of formula M-3 or a salt thereof, 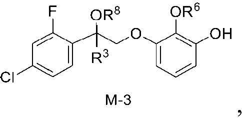 wherein R³, R⁶, and R⁸ are as defined above.

In some embodiments, the compound of formula M or a salt thereof is the compound of formula M-4 or a salt thereof, 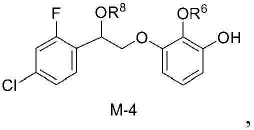 wherein R⁶ and R⁸ are as defined above.

In some embodiments, the compound of formula M or a salt thereof is the compound of formula M-4a or a salt thereof, 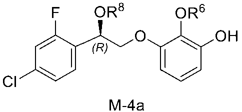 wherein R⁶ and R⁸ are as defined above.

Use of the aforementioned method for preparing the compound of formula K, the aforementioned compound of formula C, the aforementioned compound of formula D, the aforementioned compound of formula E, and the aforementioned compound of formula M in the preparation of a GLP-1 receptor agonist.

In some embodiments, the GLP-1 receptor agonist is selected from, but not limited to: 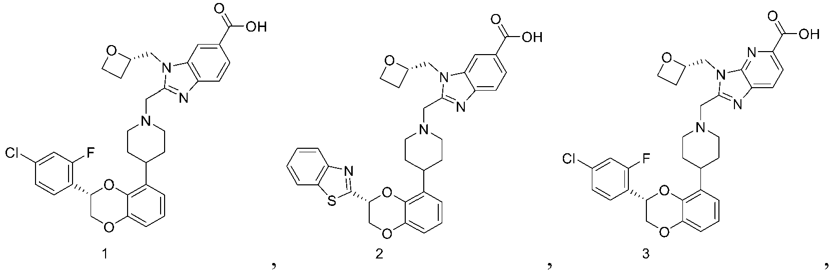 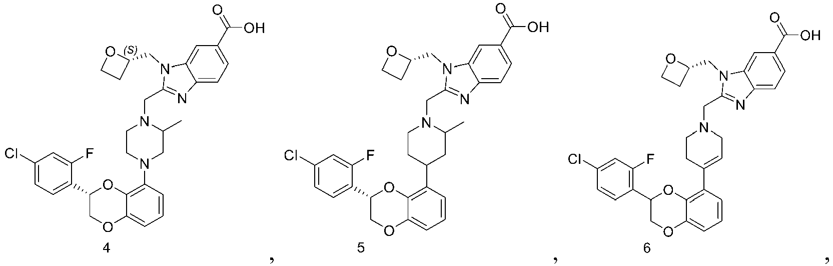 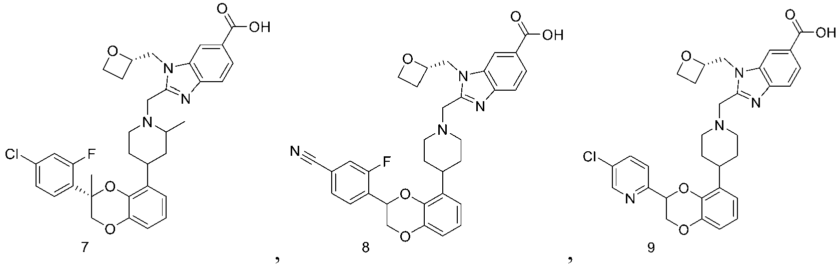 or

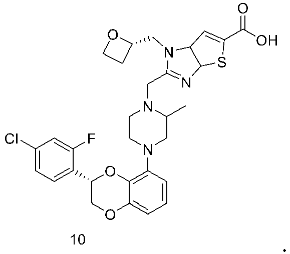

In another aspect, the present disclosure further provides a method for preparing the compound of formula 1 or a salt thereof, wherein the method comprises the steps of the aforementioned method for preparing the compound of formula F.

In certain embodiments, the method for preparing the compound of formula 1 or a salt thereof comprises the steps of the aforementioned method for preparing the compound of formula F-1. In certain embodiments, the method for preparing the compound of formula 1 or a salt thereof comprises the steps of the aforementioned method for preparing the compound of formula F-2. In certain embodiments, the method for preparing the compound of formula 1 or a salt thereof comprises the steps of the aforementioned method for preparing the compound of formula F-3. In certain embodiments, the method for preparing the compound of formula 1 or a salt thereof comprises the steps of the aforementioned method for preparing the compound of formula F-4. In certain embodiments, the method for preparing the compound of formula 1 or a salt thereof comprises the steps of the aforementioned method for preparing the compound of formula F-4a.

In certain embodiments, the method for preparing the compound of formula 1 or a salt thereof further comprises steps of converting the compound of formula F to the compound of formula G, and reacting the compound of formula G with the compound of formula H to form the compound of formula J. In certain embodiments, the method for preparing the compound of formula 1 or a salt thereof further comprises steps of converting the compound of formula F-1 to the compound of formula G-1, and reacting the compound of formula G-1 with the compound of formula H-1 to form the compound of formula J-1. In certain embodiments, the method for preparing the compound of formula 1 or a salt thereof further comprises steps of converting the compound of formula F-2 to the compound of formula G-2, and reacting the compound of formula G-2 with the compound of formula H-2 to form the compound of formula J-2. In certain embodiments, the method for preparing the compound of formula 1 or a salt thereof further comprises steps of converting the compound of formula F-3 to the compound of formula G-3, and reacting the compound of formula G-3 with the compound of formula H-3 to form the compound of formula J-3. In certain embodiments, the method for preparing the compound of formula 1 or a salt thereof further comprises steps of converting the compound of formula F-4 to the compound of formula G-4, and reacting the compound of formula G-4 with the compound of formula H-4 to form the compound of formula J-4. In certain embodiments, the method for preparing the compound of formula 1 or a salt thereof further comprises steps of converting the compound of formula F-4a to the compound of formula G-4a, and reacting the compound of formula G-4a with the compound of formula H-4a to form the compound of formula J-4a.

In certain embodiments, the method for preparing the compound of formula 1 or a salt thereof further comprises a step of converting the compound of formula J to the compound of formula K. In certain embodiments, the method for preparing the compound of formula 1 or a salt thereof further comprises a step of converting the compound of formula J-1 to the compound of formula K-1. In certain embodiments, the method for preparing the compound of formula 1 or a salt thereof further comprises a step of converting the compound of formula J-2 to the compound of formula K-2. In certain embodiments, the method for preparing the compound of formula 1 or a salt thereof further comprises a step of converting the compound of formula J-3 to the compound of formula K-3. In certain embodiments, the method for preparing the compound of formula 1 or a salt thereof further comprises a step of converting the compound of formula J-4 to the compound of formula K-4. In certain embodiments, the method for preparing the compound of formula 1 or a salt thereof further comprises a step of converting the compound of formula J-4a to the compound of formula K-4a.

In another aspect, the present disclosure further provides a method for preparing the compound of formula 1 or a salt thereof, wherein the method comprises the steps of the aforementioned method for preparing the compound of formula K.

In certain embodiments, the method for preparing the compound of formula 1 or a salt thereof comprises the steps of the aforementioned method for preparing the compound of formula K-1. In certain embodiments, the method for preparing the compound of formula 1 or a salt thereof comprises the steps of the aforementioned method for preparing the compound of formula K-2. In certain embodiments, the method for preparing the compound of formula 1 or a salt thereof comprises the steps of the aforementioned method for preparing the compound of formula K-3. In certain embodiments, the method for preparing the compound of formula 1 or a salt thereof comprises the steps of the aforementioned method for preparing the compound of formula K-4. In certain embodiments, the method for preparing the compound of formula 1 or a salt thereof comprises the steps of the aforementioned method for preparing the compound of formula K-4a.

In certain embodiments, the compound of formula 1 is the compound of formula 1a. The method for preparing the compound of formula 1a or a salt thereof further comprises a step of reacting the compound of formula K-4a with the compound of formula L to form the compound of formula 1a, 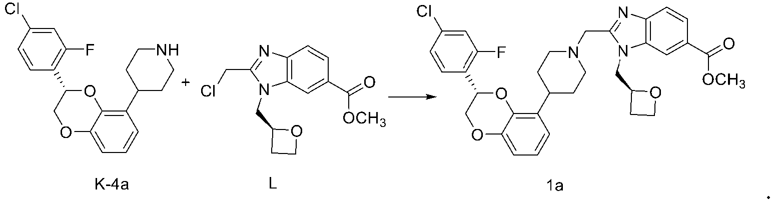 The reaction conditions and operations refer to the operations in WO2022007979, and the relevant content is incorporated herein by reference.

The preparation method of the present disclosure further comprises one or more steps selected from filtering, washing, drying, concentrating, or recrystallizing.

The salt of the compound/intermediate of the present disclosure includes, but is not limited to, an addition salt of the compound/intermediate free base form with an acid or a base, and the acid used for salt formation includes, but is not limited to, hydrochloric acid or methanesulfonic acid. In some embodiments, the salt of the compound/intermediate includes, but is not limited to, hydrochloride salt, para-toluenesulfonate salt, methanesulfonate salt, or oxalate salt.

The terms "to form" and "convert to" do not specifically indicate that the conversion reaction between two substrates is a single-step reaction; it can be a single-step or multi-step reaction between two substrates. If the intermediate contains a protecting group, the intermediate undergoes a step of removing the protecting group, and then reacts with the corresponding substrate to obtain the corresponding target product.

The numerical values in the present disclosure are instrument measurement values, and there is a certain degree of error. Generally, a range of plus or minus 10% is within a reasonable error range. Of course, the context in which the value is used needs to be considered. For example, for the particle size of the active ingredient, this value is measured with an error variation not exceeding plus or minus 10%, which can be plus or minus 9%, plus or minus 8%, plus or minus 7%, plus or minus 6%, plus or minus 5%, plus or minus 4%, plus or minus 3%, plus or minus 2%, or plus or minus 1%, preferably plus or minus 5%.

The technical solution of the present disclosure has the following beneficial effects: (1) the process steps are simple, significantly reducing production costs; (2) compared with the prior art, the reaction yield is significantly improved; (3) most prior art reports constructing chiral benzodioxane by using chiral hydroxyl to attack halogen on the benzene ring, while the present disclosure uses the Mitsunobu reaction to construct chiral benzodioxane.

In the chemical structure of the compound of the present disclosure, the bond " 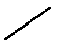 " indicates unspecified configuration, that is, if a chiral isomer exists in the chemical structure, the bond " 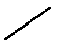 " may be " 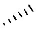 " or " 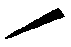 ", or may comprise both configurations of " 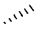 ", " and " 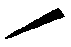 ". The bond " 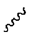 " indicates the presence of a chiral isomer in the structure, which simultaneously comprises both configurations of " 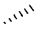 " or " 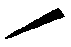 ".

The term "tautomer" or "tautomeric form" refers to structural isomers of different energies which are interconvertible via a low energy barrier. For example, prototropic tautomers (also known as proton-transfer tautomers) include tautomerization via proton migration, such as the tautomeric change between A and B as shown below in the compounds of the present disclosure.

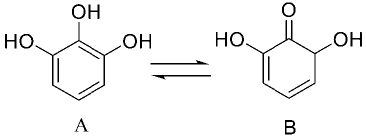

All tautomeric forms are within the scope of the present disclosure. The naming of the compound does not exclude any tautomer.

The present disclosure further includes isotopically labeled compounds of the present disclosure, which are identical to those described herein, but in which one or more atoms are replaced by atoms having an atomic weight or mass number different from the atomic weight or mass number typically found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl, respectively.

Unless otherwise specified, when a position is specifically designated as deuterium (D), that position is understood to have deuterium with an abundance of at least 1000 times greater than the natural abundance of deuterium (which is 0.015%) (i.e., at least 10% deuterium incorporation). The deuterium in the compounds of the examples having an abundance greater than the natural abundance of deuterium can be deuterium with an abundance of at least 1000 times, at least 2000 times, at least 3000 times, at least 4000 times, at least 5000 times, at least 6000 times the natural abundance of deuterium, or deuterium with a higher abundance. The present disclosure further includes various deuterated forms of the compound of formula (I). Each available hydrogen atom attached to a carbon atom can be independently replaced by a deuterium atom. Those skilled in the art can synthesize deuterated forms of the compound of formula (I) by referring to relevant literatures. The deuterated forms of the compound of formula (I) can be prepared using commercially available deuterated starting materials, or can be synthesized using deuterated reagents using conventional techniques. The deuterated reagents include, but are not limited to, deuterated borane, a solution of trideuterated borane in tetrahydrofuran, deuterated lithium aluminum hydride, deuterated iodoethane and deuterated iodomethane.

### Term explanation:

"Optionally" or "optional" refers to the fact that the subsequently described event or circumstance may, but need not, to occur, and that the description includes instances where the event or circumstance occurs and instances where it does not. For example, "C₁₋₆ alkyl optionally substituted with halogen or cyano" means that the halogen or cyano may, but need not, to be present, and that the description includes instances where the alkyl is substituted with halogen or cyano and instances where the alkyl is not substituted with halogen and cyano.

The term "alkyl" refers to a saturated aliphatic hydrocarbon group, including straight-chain and branched-chain groups having 1 to 6 carbon atoms. Non-limiting examples of alkyl include, but are not limited to: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, and various branched isomers thereof. The alkyl may be substituted or unsubstituted, and when substituted, the substituent may be substituted at any available connecting point, preferably one or more of the following groups, including but not limited to halogen, hydroxyl, cyano, or amino.

The term "alkoxy" refers to -O-(alkyl), wherein alkyl is as defined above. Non-limiting examples of alkoxy include, but are not limited to, methoxy, ethoxy, propoxy, butoxy, cyclopropoxy, cyclobutoxy, cyclopentoxy, or cyclohexoxy. The alkoxy can be optionally substituted or unsubstituted, and when substituted, the substituent is preferably one or more of the following groups, including but not limited to halogen, hydroxyl, cyano, or amino.

The term "aryl" or "aryl ring" refers to any stable, monocyclic or bicyclic carbon ring with up to 7 atoms in each ring, wherein at least one ring is aromatic. Examples of such aryl groups include, but are not limited to, phenyl, naphthyl, tetrahydronaphthyl, indanyl, biphenyl, or binaphthyl. Unless otherwise specified, an aryl or aromatic ring may be substituted or unsubstituted. When substituted, the substituent is preferably one or more of the following groups, including but not limited to halogen, cyano, C₁₋₆ alkyl, or C₁₋₆ alkoxy, wherein the alkyl or alkoxy is optionally substituted with one or more halogen, hydroxyl, cyano, or amino.

The term "heteroaryl" or "heteroaromatic ring" refers to a stable monocyclic or bicyclic ring with up to 7 atoms in each ring, wherein at least one ring is aromatic and at least one ring contains 1 to 4 heteroatoms selected from O, N, and S. Heteroaromatic rings within the scope of this definition include, but are not limited to, a pyridine ring, a thiazine ring, a pyrimidine ring, a pyridazine ring, a furan ring, a pyrrole ring, a thiophene ring, an imidazole ring, a pyrazole ring, an oxazole ring, an isoxazole ring, a thiazole ring, a benzofuran ring, an isobenzofuran ring, an isoindole ring, an indole ring, a benzothiophene ring, a benzimidazole ring, an indazole ring, a benzoxazole ring, a benzisoxazole ring, a purine ring, a benzothiazole ring, a quinoline ring, an isoquinoline ring, a quinazoline ring, a quinazolinone ring, a thioquinazolinone ring. Unless otherwise specified, a heteroaryl or heteroaromatic ring may be substituted or unsubstituted, when substituted, the substituent is preferably one or more of the following groups, including but not limited to halogen, cyano, C₁₋₆ alkyl or C₁₋₆ alkoxy, the alkyl or alkoxy is optionally substituted with one or more halogen, hydroxyl, cyano, or amino.

The term "hydroxyl protecting group" refers to a group that prevents or inhibits a hydroxyl from participating in a subsequent reaction until the protecting group is removed. Examples of hydroxyl protecting groups include, but are not limited to: acetyl, allyl, benzoyl, benzyl, β-methoxyethoxymethyl, methoxymethyl, dimethoxytrityl [bis(4-methoxyphenyl)phenylmethyl], methoxytrityl [(4-methoxyphenyl)diphenylmethyl], para-methoxybenzyl ether, methylthiomethyl, pivaloyl, tetrahydropyranyl, trityl, or silyl (e.g., trimethylsilyl, tert-butyldimethylsilyl, tert-butyldiphenylsilyl, triisopropylsilyloxymethyl, and triisopropylsilyl). Other examples include alkyl groups such as methyl and tert-butyl, and other ethers such as ethoxyethyl.

The term "amino protecting group" refers to a group that prevents or inhibits an amino group from participating in a subsequent reaction until the protecting group is removed. Examples of amino protecting groups include, but are not limited to, benzyloxycarbonyl, tert-butoxycarbonyl, 9-fluorenylmethoxycarbonyl, allyloxycarbonyl, 2-(trimethylsilyl)ethoxycarbonyl, para-toluenesulfonylmethoxycarbonyl, ethoxycarbonyl, phthaloyl, trifluoroacetyl, trityl, 2,4-dimethoxybenzyl, para-methoxybenzyl, and benzyl.

The term "halogen" refers to fluoro, chloro, bromo, or iodo.

The term "hydroxyl" refers to -OH.

The term "cyano" refers to -CN.

The term "amino" refers to -NH₂.

The term "DEAD" refers to diethyl azodicarboxylate.

The term "DIAD" refers to diisopropyl azodicarboxylate.

The term "DCAD" refers to di(4-chlorobenzyl) azodicarboxylate.

The term "DEA" refers to diethylaniline.

The term "Pd(PPh₃)₄" refers to tetrakis(triphenylphosphine)palladium.

The term "Pd(PPh₃)₂Cl₂" refers to bis(triphenylphosphine)palladium dichloride.

The term "Pd(dppe)Cl₂" refers to bis(diphenylphosphino)ethane palladium dichloride.

The term "Pd(dppp)Cl₂" refers to [1,3-bis(diphenylphosphino)propane]palladium dichloride. The term "Pd(dppf)Cl₂" refers to [1,1'-bis(diphenylphosphino)ferrocene]palladium dichloride.

The term "Pd(dppf)Cl₂·DCM" or "Pd(dppf)Cl₂·CH₂Cl₂", refers to [1,1'-bis(diphenylphosphino) ferrocene]palladium dichloride dichloromethane complex.

The term "DMF" refers to dimethylformamide.

The term "DCM" refers to dichloromethane.

The term "DIPEA" refers to N, N-diisopropylethylamine.

The term "n-BuLi" refers to n-butyllithium.

### DETAILED DESCRIPTION

The present disclosure is further described below in conjunction with examples, but these examples are not intended to limit the scope of the present disclosure.

The experimental methods not specified for the specific conditions in the examples of the present disclosure are generally carried out in accordance with conventional conditions or in accordance with the conditions recommended by the raw material or commodity manufacturer. Reagents for which specific sources are not indicated are conventional reagents purchased from the market.

The structure of the compound is determined by nuclear magnetic resonance (NMR) or/and mass spectrometry (MS). NMR chemical shift (δ) is given in units of 10⁻⁶ (ppm).

NMR measurements are performed on a Bruker AVANCE-400 spectrometer using deuterated chloroform (CDCl₃) as the solvent.

MS measurements are performed on Waters Micromass Quattro micro API triple quadrupole mass spectrometer, scanning in positive/negative ion mode, with a mass scan range of 120 to 1300.

Yantai Huanghai HSGF254 silica gel plate is used as a thin layer chromatography silica plate, and the silica gel plate for the thin layer chromatography (TLC) is of the specification of 0.2 mm±0.03 mm, and the specification when separating and purifying a product by thin layer chromatography is 0.4 mm±0.5 mm.

### Example 1: Preparation of compound 1

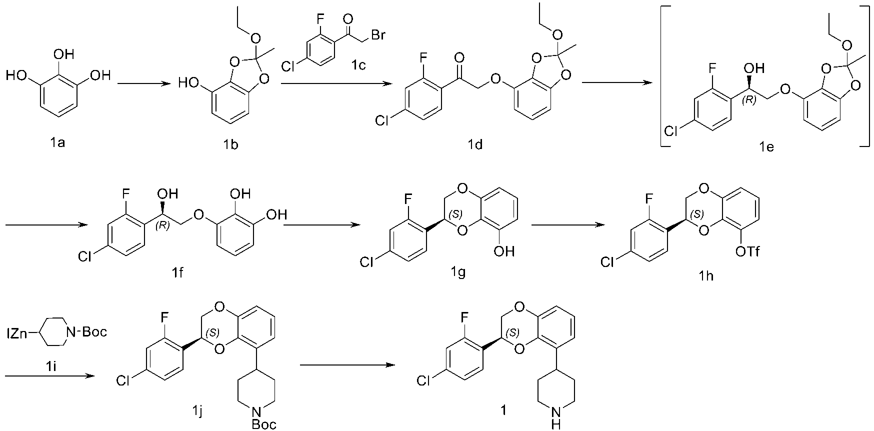

### Step 1:

To a 250 mL reaction flask were added compound 1a (40.4 g, 320 mmol, purchased from Titan Technology) and triethyl orthoacetate (78.0 g, 480 mmol, purchased from Energy Chemical), and under nitrogen protection, the mixture was heated to react until the reaction was complete. The mixture was cooled, and the solvent was removed by concentration under reduced pressure. The mixture was cooled to 0 to 10°C, and dichloromethane (160 mL) and water (40 mL) were added. The phases were separated, and the organic phase was washed with saturated sodium bicarbonate solution (40 mL × 1), water (40 mL × 2), and half-saturated sodium chloride solution (40 mL × 1), dried over anhydrous sodium sulfate, filtered to obtain compound 1b in dichloromethane, which was directly used in the next step.

¹H NMR (400 MHz, CDCl₃): δ 6.72 (t, J = 8, 1H), 6.50-6.44 (m, 2H), 5.06 (br, 1H), 3.62 (dd, J = 14.4, 7.2 Hz, 2H), 1.82 (s, 3H), 1.23-1.19 (t, 3H).

MS (ESI): m/z 197[M+H]⁺.

### Step 2:

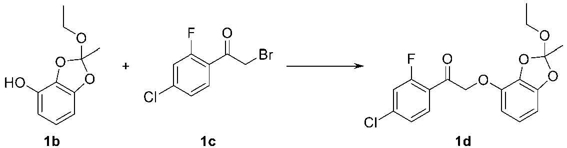

To a 250 mL reaction flask were added a solution of compound 1b (10.0 g, 50.97 mmol) in dichloromethane, potassium carbonate (9.16 g, 66.26 mmol, purchased from Sinopharm), and dichloromethane (20 mL). Under nitrogen protection, the mixture was reacted at room temperature until the reaction was complete. To the reaction system was added compound 1c (16.02 g, 63.71 mmol), and the mixture was reacted at room temperature until the reaction was complete, then filtered through a pad of diatomite, and the filter cake was washed with dichloromethane (20 mL × 2). The combined filtrate was washed with 150 mL of purified water, dried over anhydrous sodium sulfate, filtered, and the filtrate was concentrated to dryness under reduced pressure. The crude product was crystallized from n-heptane and ethanol to obtain 16.5 g of compound 1d (yield 88.3%, purity 99.2%).

¹H NMR (400 MHz, DMSO-d6): δ 7.92 (t, J = 8.4, 1H), 7.71 (dd, J = 11.2, 2 Hz, 1H), 7.49 (dd, J = 8.4, 2 Hz, 1H), 6.76 (dd, J = 8.4, 8 Hz, 1H), 6.61-6.57 (m, 2H), 5.44 (d, J = 2.8 Hz, 2H), 3.47 (dd, J = 14.4, 7.2 Hz, 2H), 1.71 (s, 3H), 1.10 (t, J = 7.2, 3H).

MS (ESI): m/z 321 [M-OEt] ⁺, 755[2M+Na] ⁺.

### Steps 3 and 4:

To a 1 L reaction flask, (R)-CBS (4.14 g, 16.36 mmol, purchased from Bide) and tetrahydrofuran (250 mL, purchased from Energy Chemical) were added. Under nitrogen protection, the temperature was controlled at 0-20°C, and borane-tetrahydrofuran complex (1 M, 340.82 mL, purchased from Energy Chemical) was added. The mixture was reacted under stirring at room temperature until the reaction was complete. A solution of compound **1d** (50 g, 136.33 mmol) in tetrahydrofuran (500 mL) was added at room temperature, and the mixture was reacted under stirring at room temperature until the reaction was complete. The temperature was controlled at 0-10°C, and 2M hydrochloric acid (250 mL) was added dropwise to quench the reaction. The mixture was stirred at room temperature until the boron complex dissociated. Tetrahydrofuran was removed under reduced pressure. The mixture was diluted with water (1000 mL), and extracted twice with EA (500 mL × 2). The organic phases were combined and washed sequentially with 2M hydrochloric acid (500 mL × 3) and brine (500 mL), dried over anhydrous sodium sulfate, filtered, and dried by rotation, and the crude product was crystallized from ethyl acetate and n-heptane to obtain 38.38 g of compound 1f (yield 94.2%, chiral purity 96.9%).

¹H NMR (400 MHz, DMSO-d6): δ 8.84 (s, 1H), 8.10 (s, 1H), 7.62 (t, J = 8.2 Hz, 1H), 7.41 (dd, J = 10.1, 2.1 Hz, 1H), 7.33 (dd, J = 8.4, 2.1 Hz, 1H), 6.52 (t, J = 8.1 Hz, 1H), 6.46 - 6.36 (m, 2H), 5.93 (d, J = 4.6 Hz, 1H), 5.18 (m, 1H), 4.06 (dd, J = 10.0, 3.5 Hz, 1H), 3.87 (dd, J = 10.1, 7.8 Hz, 1H).

MS (ESI): m/z 299[M+H] ⁺.

### Step 5:

To a 2 L reaction flask were added compound 1f (35.0 g, 1.0 eq.), triphenylphosphine (39.95 g, 1.3 eq., purchased from Titan Technology), and dichloromethane (910 mL, 26 V). The mixture was stirred evenly. Diisopropyl azodicarboxylate (30.8 g, 1.3 eq., purchased from Titan Technology) was added dropwise at 0-5°C under nitrogen protection, and the mixture was reacted under stirring until the reaction was complete. The solvent was concentrated under reduced pressure at 35°C. To the residue were added tetrahydrofuran (140 mL, 4 V) and anhydrous calcium bromide (82 g, 3.5 eq., purchased from Titan Technology). Nitrogen replacement was performed, and the mixture was reacted under stirring at room temperature until the reaction was complete. The mixture was filtered, and the filter cake was washed with tetrahydrofuran (17 mL × 2). The filtrate was concentrated under reduced pressure, dissolved in methyl tert-butyl ether (420 mL), and washed with water (140 mL × 3). The organic phase was concentrated under reduced pressure, and ethanol (35 mL) and n-heptane (245 mL) were added. The mixture was slurried at room temperature for 16 hours, filtered, and dried under reduced pressure to obtain 41.8 g of a complex (compound 1g and diisopropyl hydrazine-1,2-dicarboxylate complexed in a 2:1 ratio), with a complex yield of 127.1% (complex content about 73%, containing about 30.5 g of compound 1g, and the calculated yield of compound 1g was 92%).

¹H NMR (400 MHz, CDCl₃) δ 7.43 (t, J=8.0 Hz, 1H), 7.24-7.22 (m, 1H), 7.19-7.16 (m, 1H), 6.79 (t, J=8.4 Hz, 1H), 6.60-6.58 (m, 1H), 6.54-6.51 (m, 1H), 6.36 (br, 1H), 5.46-5.43 (m, 1H), 4.42-4.39 (m, 1H), 4.07-4.02(m, 1H).

MS (ESI): m/z 281 [M+H] ⁺, 283[M+3H] ⁺.

### Step 6:

To a 1 L reaction flask were added compound 1g (35.5 g), N,N-diisopropylethylamine (40.87 g, purchased from Titan Technology), and dichloromethane (355 mL). Under nitrogen protection, the mixture was cooled to 0-10°C, and a solution of trifluoromethanesulfonic anhydride (41.04 g, purchased from Accela) in dichloromethane (177.5 mL) was added dropwise. After the addition was completed, the mixture was reacted under stirring at 0-10°C until the reaction was complete, and then concentrated under reduced pressure at 30-35°C. The residue was dissolved in n-heptane (426 mL) and water, and the mixture was stirred evenly. The mixture was filtered through a pad of diatomite, and the filtrate was subjected to phase separation. The organic phase was washed with water (142 mL × 4), and concentrated under reduced pressure at 35°C to remove the solvent. The residue was crystallized with ethanol and water to obtain 32.2 g of compound 1h (yield 61.7%, purity 98.62%).

¹H NMR (400 MHz, CDCl₃) δ 7.52 (t, J=8.0 Hz, 1H), 7.27-7.25 (m, 1H), 7.17 (dd, J = 10, 2 Hz, 1H), 6.99-6.97 (m, 1H), 6.92 (t, J=8.4 Hz, 1H), 6.89-6.87 (m, 1H), 5.47 (dd, J = 8.8, 2.4 Hz, 1H), 4.51 (dd, J = 11.6, 2 Hz, 1H), 3.99 (dd, J = 11.6, 8.8 Hz, 1H).

### Step 7:

To a 250 mL reaction flask were added compound 1h (10.0 g, 24.23 mmol), Pd(dppf)Cl₂.CH₂Cl₂ (296.8 mg, 0.363 mmol, purchased from Leyan), and cuprous iodide (276.8 mg, 1.45 mmol, purchased from Energy Chemical). Argon replacement was performed. Under argon protection, a solution of compound 1i in N,N-dimethylacetamide (44 mL, 48.46 mmol, purchased from WuXi AppTec) was added, and the mixture was heated to react until the reaction was complete. The reaction solution was cooled to 35°C. The reaction solution was quenched by adding a saturated ammonium chloride solution (100 mL), stirred for 20 minutes, extracted with ethyl acetate, dried over anhydrous sodium sulfate, concentrated under reduced pressure, and purified by column chromatography (n-heptane:ethyl acetate = 20:1-50:1) to obtain 13.5 g of compound 1j (yield 124.4%, purity 85.3%).

¹HNMR (400 MHz, CDCl₃): δ 7.41(t, J=8.0 Hz, 1H), 7.23 (dd, J = 8.4, 1.6 Hz, 1H), δ 7.16(dd, J = 10, 2 Hz, 1H), δ 6.87-6.77(m, 3H), 5.42(dd, J =8.4, 2.4Hz, 1H), 4.41(dd, J =11.6, 2.4Hz, 1H), δ 4.23-4.11(m, 2H), 3.97(dd, J =11.2, 8.4Hz, 1H), 3.09-3.03(m, 1H), 2.84-2.74(m, 2H), 1.88-1.77(m, 2H), 1.66-1.60(m, 2H), 1.47(s, 9H)
MS (ESI): m/z 917[2M+Na] ⁺

Step 8:

To a 250 mL reaction flask were added compound 1j (13.5 g, 24.23 mmol) and dioxane (10 mL). The temperature was controlled below 30°C, and 4M hydrochloric acid dioxane (50 mL) was added under stirring. The mixture was reacted at room temperature until the reaction was complete, and concentrated to dryness under reduced pressure. Tetrahydrofuran (45 mL) was added, followed by dropwise addition of isopropyl ether (215 mL), and the mixture was stirred for 1 hour. The mixture was filtered under suction, and the filter cake was washed with isopropyl ether (8 mL). The filter cake was added to ethyl acetate (110 mL) and a 5% potassium carbonate solution (55 mL), and stirred for 20 minutes. The phases were separated, and the aqueous phase was extracted with ethyl acetate (40 mL × 1). The organic phases were combined, washed with purified water (75 mL × 2), dried over anhydrous sodium sulfate, filtered, concentrated to dryness under reduced pressure to obtain 7.05 g of compound 1 (yield 67.2%, chiral purity 100%).

¹HNMR (400 MHz, CDCl3): δ 7.43 (t, J=8Hz, 1H), 7.24 (dd, J = 8.4, 2 Hz, 1H), 7.16 (dd, J =10, 1.6 Hz, 1H), 6.90-6.79(m,3H), 5.41(dd, J =8, 2 Hz, 1H), 4.41(dd, J =11.2, 2.4 Hz, 1H), 3.99-3.94(m,1H), 3.20-3.15(m,2H), 3.09-3.03(m,1H) , 2.80-2.69(m,2H) , 1.90-1.86(m,1H) , 1.82-1.77(m,1H), 1.68-1.61(m,2H)
MS (ESI): m/z 348[M+H] ⁺.

## Claims

1. A method for preparing a compound of formula K-1 or a salt thereof, wherein 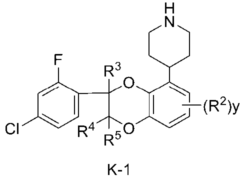 the method comprises a step of reacting a compound of formula E-1 in the presence of trisubstituted phosphine/azodicarboxylate to form a compound of formula F-1, 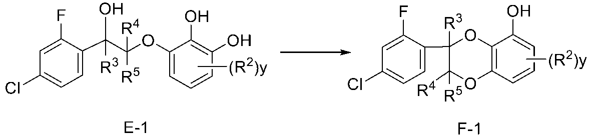 wherein,
R² is selected from halogen, cyano, C₁₋₆ alkyl or C₁₋₆ alkoxy;
R³, R⁴, and R⁵ are each independently selected from hydrogen, halogen, cyano, C₁₋₆ alkyl or C₁₋₆ alkoxy, wherein the alkyl or alkoxy is optionally substituted with one or more halogen, hydroxyl, cyano, or amino;
y is selected from 0, 1, 2, or 3.

2. The method according to claim 1, wherein the trisubstituted phosphine is selected from triphenylphosphine.

3. The method according to claim 1 or 2, wherein the azodicarboxylate is selected from diethyl azodicarboxylate, diisopropyl azodicarboxylate, and di(4-chlorobenzyl) azodicarboxylate, preferably diisopropyl azodicarboxylate.

4. The method according to any one of claims 1 to 3, further comprising a step of converting a compound of formula D-1 to a compound of formula M-1 in the presence of an acid, and a step of converting the compound of formula M-1 to the compound of formula E-1, 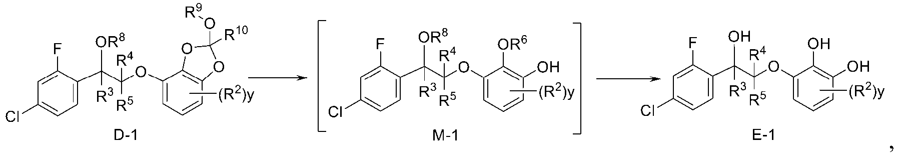 wherein,
R⁶ and R⁸ are each independently selected from hydrogen or a hydroxyl protecting group;
R⁹ and R¹⁰ are each independently selected from hydrogen, C₁₋₆ alkyl or C₁₋₆ alkoxy, wherein the alkyl or alkoxy is optionally substituted with one or more halogen, hydroxyl, cyano, or amino;
R², R³, R⁴, R⁵, and y are as defined in claim 1.

5. The method according to claim 4, wherein R⁶ is selected from hydrogen, allyl, methoxymethyl ether, tetrahydropyranyl, (trimethylsilyl)ethoxymethyl, dimethylaminosulfonyl, trifluoromethanesulfonyl, methyl, tert-butyl, trityl, benzyl, para-methoxybenzyl, tert-butyldimethylsilyl, acetyl, benzoyl, and benzyloxycarbonyl, preferably hydrogen.

6. The method according to any one of claims 1 to 5, further comprising a step of forming the compound of formula D-1 from a compound of formula C-1 in the presence of an oxazaborolidine catalyst of formula (I)/a reducing agent, 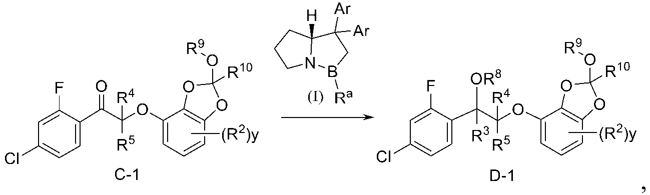 wherein,
each Ar is independently selected from 6-membered aryl or 5- to 10-membered heteroaryl, wherein the aryl or heteroaryl is optionally substituted with one or more halogen, cyano, C₁₋₆ alkyl, or C₁₋₆ alkoxy;
R^{a} is selected from hydrogen or C₁₋₆ alkyl, wherein the alkyl is optionally substituted with one or more halogen, hydroxyl, cyano, or amino;
R², R³, R⁴, R⁵, and y are as defined in claim 1, and R⁸, R⁹, and R¹⁰ are as defined in claim 4.

7. The method according to claim 6, wherein the oxazaborolidine catalyst of formula (I) is represented by formula (Ia)

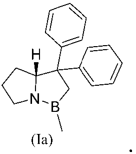

8. The method according to claim 6 or 7, wherein the reducing agent is a borane-ligand compound, preferably BH₃ -THF, BH₃·Me₂S, BH₃·DEA, and most preferably BH₃·THF.

9. The method according to any one of claims 1 to 8, further comprising steps of reacting the compound of formula (II) with an orthoester of formula (III) to form a compound of formula A, and reacting the compound of formula A with a compound of formula B1 in the presence of a base to form the compound of formula C-1, 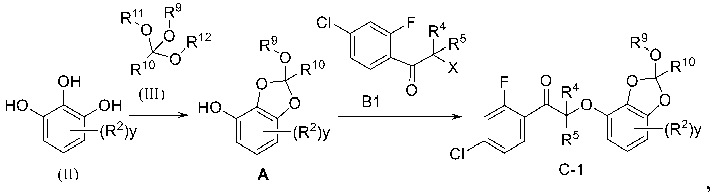 wherein,
R¹¹ and R¹² are each independently selected from hydrogen, C₁₋₆ alkyl or C₁₋₆ alkoxy, wherein the alkyl or alkoxy is optionally substituted with one or more halogen, hydroxyl, cyano, or amino;
X is halogen;
R², R⁴, R⁵, and y are as defined in claim 1, and R⁹ and R¹⁰ are as defined in claim 4.

10. The method according to claim 9, wherein the base is selected from potassium carbonate or sodium carbonate, preferably potassium carbonate.

11. The method according to claim 9 or 10, wherein R¹¹ and R¹² are each independently C₁₋₆ alkyl, preferably methyl, ethyl, propyl, butyl, isopropyl, and most preferably ethyl.

12. The method according to any one of claims 4 to 11, wherein R⁹ and R¹⁰ are each independently selected from hydrogen, methyl, ethyl, propyl, butyl, or isopropyl, preferably methyl or ethyl.

13. The method according to any one of claims 1 to 12, further comprising steps of converting the compound of formula F-1 to a compound of formula G-1, and reacting the compound of formula G-1 with a compound of formula H-1 in the presence of a palladium catalyst/a co-catalyst to form a compound of formula J-1, 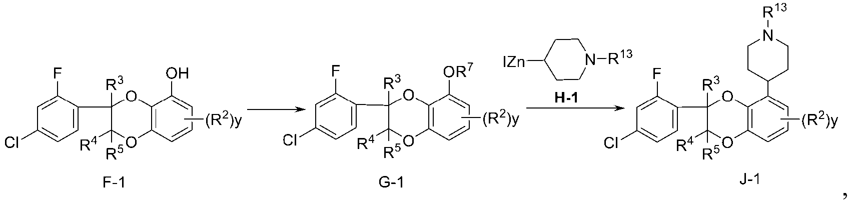 wherein,
R⁷ is a hydroxyl protecting group;
R¹³ is an amino protecting group;
R², R³, R⁴, R⁵, and y are as defined in claim 1.

14. The method according to claim 13, wherein R⁷ is selected from allyl, methoxymethyl ether, tetrahydropyranyl, (trimethylsilyl)ethoxymethyl, dimethylaminosulfonyl, trifluoromethanesulfonyl, methyl, tert-butyl, trityl, benzyl, para-methoxybenzyl, tert-butyldimethylsilyl, acetyl, benzoyl, and benzyloxycarbonyl, preferably trifluoromethanesulfonyl.

15. The method according to claim 13 or 14, wherein the palladium catalyst is selected from Pd(PPh₃)₄, Pd(PPh₃)₂Cl₂, Pd(dppe)Cl₂, Pd(dppp)Cl₂, Pd(dppf)Cl₂, a solvate of Pd(PPh₃)₄, a solvate of Pd(PPh₃)₂Cl₂, a solvate of Pd(dppe)Cl₂, a solvate of Pd(dppp)Cl₂, a solvate of Pd(dppf)Cl₂, preferably Pd(dppf)Cl₂ or a solvate of Pd(dppf)Cl₂.

16. The method according to any one of claims 13 to 15, wherein the co-catalyst is selected from silver oxide or cuprous iodide.

17. The method according to any one of claims 1 to 16, further comprising a step of converting the compound of formula J-1 to the compound of formula K-1 in the presence of hydrochloric acid/dioxane, 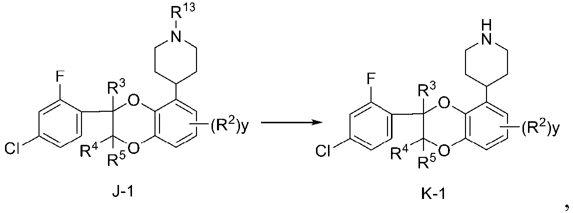 R², R³, R⁴, R⁵, and y are as defined in claim 1, and R¹³ is as defined in claim 13.

18. The method according to any one of claims 13 to 17, wherein R¹³ is selected from benzyloxycarbonyl, tert-butoxycarbonyl, 9-fluorenylmethoxycarbonyl, allyloxycarbonyl, 2-(trimethylsilyl)ethoxycarbonyl, para-toluenesulfonylmethoxycarbonyl, ethoxycarbonyl, phthaloyl, trifluoroacetyl, trityl, 2,4-dimethoxybenzyl, para-methoxybenzyl, and benzyl, preferably tert-butoxycarbonyl.

19. The method according to any one of claims 1 to 18, wherein y is 0.

20. The method according to any one of claims 1 to 19, wherein R⁴ and R⁵ are each independently hydrogen.

21. The method according to any one of claims 1-8 and 13-20, wherein R³ is selected from hydrogen, methyl, ethyl, propyl, butyl, fluoro, chloro, bromo, and cyano, preferably hydrogen and methyl.

22. The method according to any one of claims 1-21, wherein the compound of formula K or formula K-1 is a compound of formula K-4a,

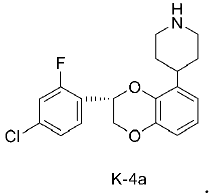

23. A method for preparing the compound of formula K-4a or a salt thereof, comprising:
step 1) reacting a compound of formula (IIa) with an orthoester of formula (IIIa) to form a compound of formula A2, and reacting the compound of formula A2 with a compound of formula B3 in the presence of potassium carbonate to form a compound of formula C-4;
step 2) forming a compound of formula D-4a from the compound of C-4 in the presence of the oxazaborolidine catalyst of formula (Ia)/BH₃·THF;
step 3) reacting the compound of formula D-4a in the presence of hydrochloric acid/methanol to form a compound of formula E-4a
step 4) a step of reacting the compound of formula E-4a in the presence of triphenylphosphine/diisopropyl azodicarboxylate to form a compound of formula F-4a,
step 5) converting the compound of formula F-4a to a compound of formula G-4a, and reacting the compound of formula G-4a with a compound of formula H-2 in the presence of Pd(dppf)Cl₂ or a solvate of Pd(dppf)Cl₂ and cuprous iodide to form a compound of formula J-4a; and
step 6) converting the compound of formula J-4a to the compound of formula K-4a in the presence of hydrochloric acid/dioxane; 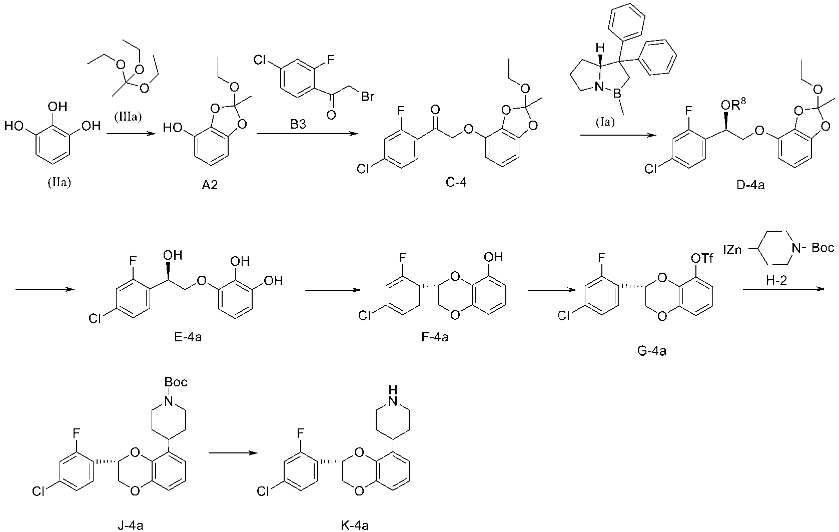 wherein R⁸ is as defined in claim 4.

24. The method according to claim 15 or 23, wherein the solvate of Pd(dppf)Cl₂ is Pd(dppf)Cl₂·CH₂Cl₂.

25. The method according to any one of claims 4 and 23-24, wherein R⁸ is selected from hydrogen, allyl, methoxymethyl ether, tetrahydropyranyl, (trimethylsilyl)ethoxymethyl, dimethylaminosulfonyl, trifluoromethanesulfonyl, methyl, tert-butyl, trityl, benzyl, para-methoxybenzyl, tert-butyldimethylsilyl, acetyl, benzoyl, and benzyloxycarbonyl, preferably hydrogen.

26. A compound of formula C or a salt thereof, 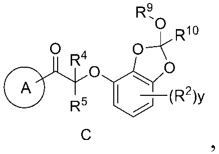 wherein,
ring A is selected from 6- to 10-membered aryl or 5- to 10-membered heteroaryl, wherein the aryl or heteroaryl is optionally substituted with one or more halogen, cyano, C₁₋₆ alkyl or C₁₋₆ alkoxy;
R² is selected from halogen, cyano, C₁₋₆ alkyl or C₁₋₆ alkoxy;
R⁴ and R⁵ are each independently selected from hydrogen, halogen, cyano, C₁₋₆ alkyl or C₁₋₆ alkoxy, wherein the alkyl or alkoxy is optionally substituted with one or more halogen, hydroxyl, cyano, or amino;
R⁹ and R¹⁰ are each independently selected from hydrogen, C₁₋₆ alkyl or C₁₋₆ alkoxy, wherein the alkyl or alkoxy is optionally substituted with one or more halogen, hydroxyl, cyano, or amino;
y is selected from 0, 1, 2, or 3;
further, the compound of formula C is preferably a compound of formula C-1 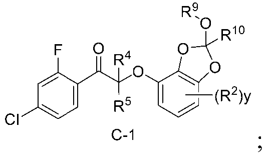 and
further, the compound of formula C is preferably a compound of formula C-4

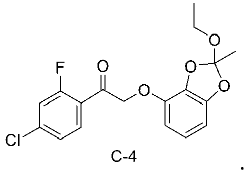

27. A compound of formula D or a salt thereof, 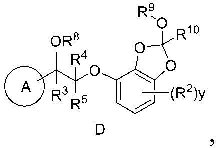 wherein,
R³ is selected from hydrogen, halogen, cyano, C₁₋₆ alkyl or C₁₋₆ alkoxy, wherein the alkyl or alkoxy is optionally substituted with one or more halogen, hydroxyl, cyano, or amino;
R⁸ is selected from hydrogen or a hydroxyl protecting group;
ring A, R², R⁴, R⁵, R⁹, R¹⁰, and y are as defined in claim 26;
further, the compound of formula D is preferably a compound of formula D-1 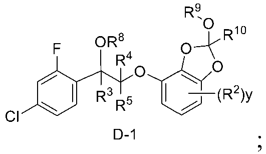 and
further, the compound of formula D is preferably a compound of formula D-4a

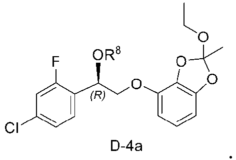

28. A compound of formula E or a salt thereof, 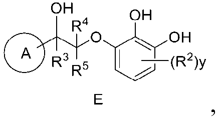
wherein ring A, R², R⁴, R⁵, and y are as defined in claim 26, and R³ is as defined in claim 27;
further, the compound of formula E is preferably a compound of formula E-1 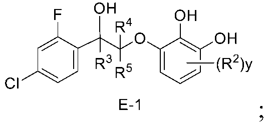 and
further, the compound of formula E is preferably a compound of formula E-4a

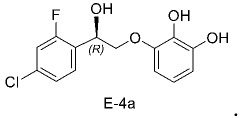

29. Use of the method according to any one of claims 1 to 25, the compound of formula C according to claim 26, the compound of formula D according to claim 27, and the compound of formula E according to claim 28 in the preparation of a GLP-1 receptor agonist.

30. A method for preparing a compound of formula 1 or a salt thereof, wherein the method comprises the steps of the method according to any one of claims 1 to 25,
